# EUROPEAN PATENT SPECIFICATION

(11) **EP 2 235 535 B1**
(45) Date of publication and mention of the grant of the patent: **12.11.2014**
(21) Application number: 08859508.7
(22) Date of filing: 05.12.2008
(51) Int. Cl.: G01N 33/574, C12Q 1/68

(54) **BIOMARKERS FOR PREDICTING THE SENSITIVITY OF CELLS TO IMMUNOMODULATORY COMPOUNDS DURING TREATMENT OF NON-HODGKIN'S LYMPHOMA**
BIOMARKER ZUR VORHERSAGE DER EMPFINDLICHKEIT VON ZELLEN GEGENÜBER IMMUNMODULATORISCHEN VERBINDUNGEN WÄHREND DER BEHANDLUNG VON NON-HODGKIN-LYMPHOM
BIOMARQUEURS POUR PRÉDIRE LA SENSIBILITÉ DE CELLULES À DES COMPOSÉS IMMUNORÉGULATEURS PENDANT UN TRAITEMENT DE LYMPHOME NON HODGKINIEN

(30) Priority: 07.12.2007 US 5806 P
(43) Date of publication of application: 06.10.2010
(73) Proprietor: CELGENE CORPORATION, Summit, NJ 07901 (US)
(72) Inventor: SCHAFER, Peter, H., Somerset, NJ 08873 (US); BARTLETT, Justin, B., Warren NJ 09059 (US); ZHANG, Ling-Hua, Parsippany NJ 07054 (US)
(74) Representative: Savic, Bojan
(86) International application number: PCT/US2008/013448
(87) International publication number: WO 2009/075797

(56) References cited:
- US-A1- 2006 041 387
- US-A1- 2006 135 423
- US-A1- 2007 117 133
- PELLAGATTI A ET AL: "Lenalidomide inhibits the malignant clone and up-regulates the SPARC gene mapping to the commonly deleted region in 5q- syndrome patients." PROC NAT ACAD SCI USA, vol. 104, no. 27, 3 July 2007 (2007-07-03), pages 11406-11411, XP002519682
- WITZIG T E ET AL: "Initial results from an international study in relapsed/refractory aggressive non-Hodgkin's lymphoma to confirm the activity, safety and criteria for predicting response to lenalidomide monotherapy" BLOOD, vol. 110, no. 11, Part 1, November 2007 (2007-11), page 758A, XP002519683 & 49TH ANN MEETING OF THE AM SOC HEMATOL; ATLANTA, GA, USA; DEC 08 -11, 2007
- CANIONI D ET AL: "Immunohistochemical markers can be of predictive value in Hodgkin's lymphoma response to treatment. A preliminary study of 23 cases" BLOOD, vol. 104, no. 11, Part 1, November 2004 (2004-11), page 853A, XP002519684 & 46TH ANN MEETING OF THE AM SOC HEMATOL; SAN DIEGO, CA, USA; DEC 04 -07, 2004
- ZHANG L-H ET AL: "Lenalidomide displays direct anti-non-Hodgkin's lymphoma (NHL) cell activity in association with enhanced SPARC expression but independent of its ability to strongly inhibit NHL cell VEGF production In Vitro" BLOOD, vol. 110, no. 11, Part 1, November 2007 (2007-11), pages 1017A-1018A, XP002519685 & 49TH ANN MEETING OF THE AM SOC HEMATOL; ATLANTA, GA, USA; DEC 08 -11, 2007
- FUKUNAGA-KALABIS M ET AL: "Unraveling mysteries of the multifunctional protein SPARC." J INVESTIG DERMATOL, vol. 127, no. 11, November 2007 (2007-11), pages 2497-2498, XP002519686
- BOND W S ET AL: "Detection methods and strategies for improving medication compliance." AM J HOSP PHARMACY, vol. 48, no. 9, September 1991 (1991-09), pages 1978-1988, XP009113936
- DATABASE BIOSIS [Online] BIOSCIENCES INFORMATION SERVICE, US; 16 November 2002 (2002-11-16), DUCKWORTH A W ET AL: "Cyclin D1 mRNA Transcript Quantification in Peripheral Blood of Mantle Cell Lymphoma Patients: A Robust Diagnostic Test." XP002530817 Database accession no. PREV200300368139 & BLOOD, vol. 100, no. 11, 16 November 2002 (2002-11-16), page Abstract No. 4655, 44TH ANNUAL MEETING OF THE AMERICAN SOCIETY OF HEMATOLOGY; PHILADELPHIA, PA, USA; DECEMBER 06-10, 2002 ISSN: 0006-4971 -& SUZUKI R ET AL: "Detection of Cyclin D1 Overexpression by Real-Time Reverse-Transcriptase-Mediated Quantitative Polymerase Chain Reaction for the Diagnosis of Mantle Cell Lymphoma" AM J PATHOL, vol. 159, no. 2, 1 August 2001 (2001-08-01), pages 425-429, XP002993187
- BIJWAARD K E ET AL: "Quantitative real-time reverse transcription-PCR assay for cyclin DI expression: Utility in the diagnosis of mantle cell lymphoma" CLIN CHEM, vol. 47, no. 2, February 2000 (2000-02), pages 195-201, XP002530816
- DATABASE MEDLINE [Online] US NATIONAL LIBRARY OF MEDICINE (NLM); September 2004 (2004-09), AKERVALL J ET AL: "Overexpression of cyclin D1 correlates with sensitivity to cisplatin in squamous cell carcinoma cell lines of the head and neck." XP002530818 Database accession no. NLM15484403 & ACTA OTO-LARYNGOLOGICA, vol. 124, no. 7, September 2004 (2004-09), pages 851-857,
- ROBINSON M D ET AL: "A comparison of Affymetrix gene expression arrays" BMC BIOINFORMATICS, vol. 8, no. 1, 15 November 2007 (2007-11-15), page 449, XP021031592

## Description

### 1. FIELD

Provided herein is monitoring of expression of a specific set of genes or proteins before and during therapy with an immunomodulatory compound to treat cancer, *e.g*., non-Hodgkin's lymphoma patients.

### 2. BACKGROUND

Various compounds have been used to treat cancer, and among these compounds that are capable of modulating the immune system. Some studies have focused on a group of immunomodulatory compounds that were initially selected for their capacity to potently inhibit TNF-α production by LPS stimulated PBMC. L.G. Corral, et al., Ann. Rheum. Dis., 58 (suppl I): 1107-1113 (1999). Celgene Corporation's immunomodulatory compounds, referred to as IMiDs^{®}, show not only potent inhibition of TNF-α but also marked inhibition of LPS induced monocyte IL1ß and IL 12 production. Particular examples of immunomodulatory compounds include, but are not limited to, the substituted 2-(2,6-dioxopiperidin-3-yl) phthalimides and substituted 2-(2,6-dioxopiperidin-3-yl)-1-oxoisoindoles described and claimed in United States Patent Nos. 6,281,230 and 6,316,471, both to G.W. Muller, et al*.*

One of these compounds, 1-oxo-2-(2,6-dioxopiperidin-3-yl)-4-aminoisoindoline, is an anti-angiogenic, anti-proliferative and immunomodulatory drug that is approved for the treatment of transfusion-dependent patients with anemia due to low- or intermediate-risk MDS associated with a del 5q cytogenetic abnormality with or without additional cytogenetic abnormalities. 1-oxo-2-(2,6-dioxopiperidin-3-yl)-4-aminoisoindoline is also approved for use in combination with dexamethasone for the treatment of previously treated multiple myeloma patients.

US 2007/117133 provides a method for predicting or determining the response of a mammalian tumor to a chemotherapeutic agent and for treating a mammalian tumor comprising detecting and quantifying the SPARC protein or RNA in a sample isolated from the mammal. Document PELLAGATTI et al, PNAS USA, 104(27), 2007, 11406-11411 discloses that lenalidomide inhibits the malignant clone and up-regulates the SPARC gene mapping to the commonly deleted region in 5q-syndrome patients. ZHANG et al, BLOOD, 110 (11 Part 1), 2007, 1017A-1018A discloses that lenalidomide displays direct Anti-Non-Hodgkin's Lymphoma (NHL) cell activity in association with enhanced SPARC expression but independent of its ability to strongly inhibit NHL cell VEGF production *in vitro.* ROBINSON et al, BMC BIOINFORMATICS, 8(1), 2007, 449 refers to probe-, exon- and gene-level reproducibility of technical and biological replicates from each of the 3 platforms. US 2006/041387 refers to cancer detection and, in particular, to a system for use in early diagnosis of cancer using high-throughput microarray technology. US 2006/135423 relates to methods and compositions for the treatment or prevention of ocular angiogenesis and neovascularization associated with neovascular disease.

### 3. SUMMARY

Provided herein is the use of specific mRNAs and proteins as biomarkers to ascertain the effectiveness and progress of the treatment by immunomodulatory compounds. For example, the mRNA or protein levels of SPARC, p21, and cyclin D1 can be used to determine whether an immunomodulatory compound, such as 1-oxy-2-(2,6-dioxopiperidin-3-yl)-4-aminoisoindoline, is likely to be successful in treating certain types of cancer, such as NHL. Further, the expression of these genes or proteins can be used to monitor progress of treatment effectiveness in NHL patients that are receiving treatment with immunomodulatory compounds.

In some embodiments, a method of predicting tumor response to treatment in a Non-Hodgkin's Lymphoma (NHL) patient is provided. The method comprises obtaining tumor cells from the patient, culturing the cells in the presence or absence of an immunomodulatory compound, measuring SPARC expression in the tumor cells, and comparing the levels of SPARC expression level in tumor cells cultured in the presence of an immunomodulatory compound to those in tumor cells cultured in the absence of an immunomodulatory compound, wherein an increased level of SPARC expression in the presence of an immunomodulatory compound indicates the likelihood of an effective patient tumor response to the immunomodulatory compound.

In another embodiment, a method of monitoring tumor response to treatment in a Non-Hodgkin's Lymphoma (NHL) patient is provided. The method comprises obtaining a biological sample from the patient, measuring SPARC expression in the biological sample, administering an immunomodulatory compound to the patient, thereafter obtaining a second biological sample from the patient, measuring SPARC expression in the second biological sample, and comparing the levels of SPARC expression, where an increased level of SPARC expression after treatment indicates the likelihood of an effective tumor response.

In yet another embodiment, a method for monitoring patient compliance with a drug treatment protocol is provided. The method comprises obtaining a biological sample from the patient, measuring the expression level of at least one of p21, or SPARC in the sample, and determining if the expression level is increased or decreased in the patient sample compared to the expression level in a control untreated sample, wherein an increased or decreased expression indicates patient compliance with the drug treatment protocol. In one embodiment, the expression of p21 or SPARC is increased.

The expression monitored can be, for example, mRNA expression or protein expression. The expression in the treated sample can increase, for example, by about 1.5X, 2.0X, 3X, 5X, or more. The immunomodulatory compound is 1,3-dioxo-2-(2,6-dioxopiperidin-3-yl)-4-aminoisoindoline.

Disclosed herein is a method of predicting the sensitivity to treatment with an immunomodulatory compound in an NHL, specifically, a Mantle Cell Lymphoma (MCL), patient is provided. The method comprises obtaining a biological sample from the patient, optionally isolating or purifying mRNA from the biological sample, amplifying the mRNA transcripts by, *e.g.,* RT-PCR, where a higher baseline level of Cyclin D1 (as assessed by, *e.g.,* determining the cycle number at which the fluorescence passes the set threshold level ("CT") of Cyclin D1 mRNA expression) indicates a higher likelihood that the cancer will be sensitive to treatment with an immunomodulatory compound.

Also disclosed is a kit useful for predicting the likelihood of an effective treatment of NHL with an immunomodulatory compound is provided. The kit comprises a solid support, nucleic acids contacting the support, where the nucleic acids are complementary to at least 20, 50, 100, 200, 350, or more bases of cyclin D1 mRNA, and a means for detecting the expression of the mRNA in a biological sample.

In an additional embodiment, a kit useful for predicting the likelihood of an effective NHL treatment or for monitoring the effectiveness of a treatment with an immunomodulatory compound is provided. The kit comprises a solid support, at least one nucleic acid contacting the support, where the nucleic acid is complementary to at least 20, 50, 100, 200, 350, 500, or more bases of SPARC mRNA, and a means for detecting the expression of the mRNA in a biological sample.

In an additional embodiment, a kit useful for predicting the likelihood of an effective treatment of NHL or for monitoring treatment with an immunomodulatory compound is provided. The kit comprises a solid support, and a means for detecting the protein expression of at least one of SPARC, and p21 in a biological sample.

Such a kit can employ, for example a dipstick, a membrane, a chip, a disk, a test strip, a filter, a microsphere, a slide, a multiwell plate, or an optical fiber. The solid support of the kit can be, for example, a plastic, silicon, a metal, a resin, glass, a membrane, a particle, a precipitate, a gel, a polymer, a sheet, a sphere, a polysaccharide, a capillary, a film, a plate, or a slide. The biological sample can be, for example, a cell culture, a cell line, a tissue, an oral tissue, gastrointestinal tissue, an organ, an organelle, a biological fluid, a blood sample, a urine sample, or a skin sample. The biological sample can be, for example, a lymph node biopsy, a bone marrow biopsy, or a sample of peripheral blood tumor cells.

### 4. BRIEF DESCRIPTION OF THE FIGURES

**FIG. 1** is a bar graph illustrating the effect of immunomodulatory compounds on VEGF, p21, p53, and cyclin D1 gene expression in Rec-1 cells. The cells were treated with either DMSO (control), 1, 10, or 100 µM of immunomodulatory compound, dexamethasone (10 nM), or a combination of the immunomodulatory compound plus dexamethasone as indicated. The fold change in mRNA expression level is shown.
**FIG. 1A** illustrates the change in gene expression after incubation with the immunomodulatory compound 1,3-dioxo-2-(2,6-dioxopiperidin-3-yl)-4-aminoisoindoline for 24 hours.
**FIG. 1B** illustrates the change in gene expression after incubation with the immunomodulatory compound 1,3-dioxo-2-(2,6-dioxopiperidin-3-yl)-4-aminoisoindoline for 48 hours.
**FIG. 1C** illustrates the change in gene expression after incubation with the immunomodulatory compound 1-oxo-2-(2,6-dioxopiperidin-3-yl)-4-aminoisoindoline for 24 hours.
**FIG. 1D** illustrates the change in gene expression after incubation with the immunomodulatory compound 1-oxo-2-(2,6-dioxopiperidin-3-yl)-4-aminoisoindoline for 48 hours.
**FIG. 2** is a bar graph illustrating the fold change in gene expression of p21, Activin A, or SPARC in Jeko-1 cells after 24 hours of incubation with DMSO (control), 1,3-dioxo-2-(2,6-dioxopiperidin-3-yl)-4-aminoisoindoline (1 µM or 10 µM), 1-oxy-2-(2,6-dioxopiperidin-3-yl)-4-aminoisoindoline (1 µM or 10 µM), dexamethasone (10 nM), or a combination of either 1,3-dioxo-2-(2,6-dioxopiperidin-3-yl)-4-aminoisoindoline (10 µM) or 1-oxo-2-(2,6-dioxopiperidin-3-yl)-4-aminoisoindoline (10 µM) plus dexamethasone (10 nM).
**FIG. 3** illustrates that the sensitivity of a specific Mantle Cell Lymphoma cell line (Rec-1, Jeko-1, Granta-519, or JVM-2) to the immunomodulatory compound 1-oxo-2-(2,6-dioxopiperidin-3-yl)-4-aminoisoindoline correlates with a baseline level *(i.e.,* the level prior to treatment) of high cyclin D1 gene expression.
**FIG. 4** is a set of bar graphs comparing the effect of the immunomodulatory compound 1-oxo-2-(2,6-dioxopiperidin-3-yl)-4-aminoisoindoline on cell proliferation and SPARC expression in various cell lines.
**FIG. 4A** demonstrates the effect of a 3 day incubation with 1-oxo-2-(2,6-dioxopiperidin-3-yl)-4-aminoisoindoline (at 0.1 µM, 1 µM, 10 µM, or 100 µM) on the inhibition of cell proliferation in various cell lines. The results (by cell line) are sorted (left to right) from most sensitive to least sensitive to 1-oxo-2-(2,6-dioxopiperidin-3-yl)-4-aminoisoindoline.
**FIG. 4B** demonstrates SPARC gene expression (in relative units) after a 1 day incubation with the immunomodulatory compound 1-oxo-2-(2,6-dioxopiperidin-3-yl)-4-aminoisoindoline (at 1 µM or 10 µM). The results are sorted by cell line, from the highest (left) to lowest (right) increase in relative SPARC expression.

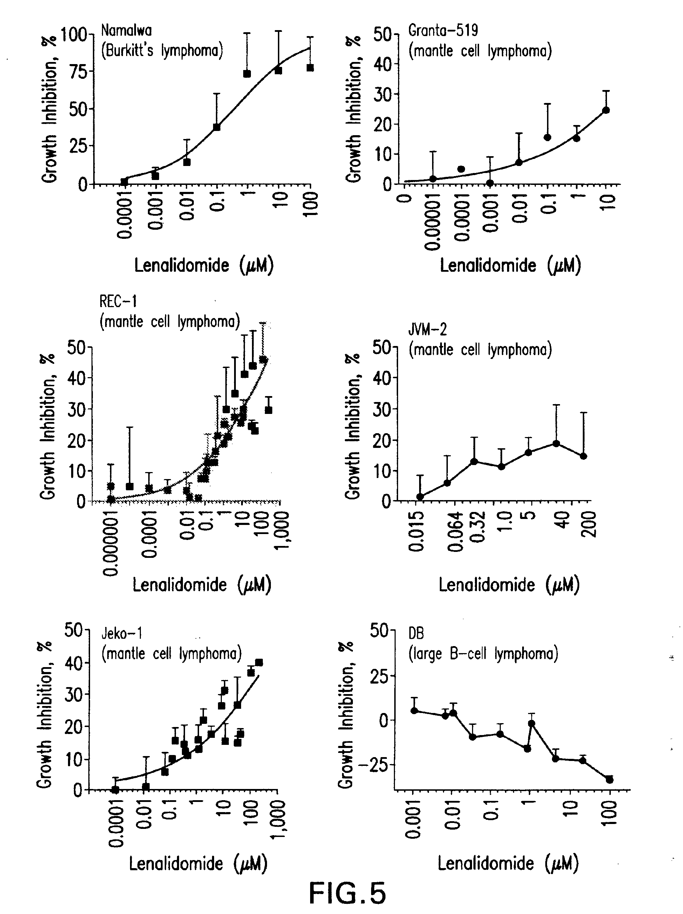

**FIG. 5** is a set of line graphs demonstrating the effect of 1-oxo-2-(2,6-dioxopiperidin-3-yl)-4-aminoisoindoline alone on the proliferation of various NHL cells (n = 2-6): **FIG. 5A:** Namalwa; **FIG. 5B:** Granta-519; **FIG. 5C:** REC-1; **FIG. 5D:** JVM-2; **FIG. 5E:** Keko-1; and **FIG. 5F:** DB.
**FIG. 6** is a pair of line graphs demonstrating that the synergy between 1-oxo-2-(2,6-dioxopiperidin-3-yl)-4-aminoisoindoline and dexamethasone reduces Jeko-1 cell viability by arresting cell cycle in G0/G1 phrase **(****FIG. 6A****)** and promoting apoptosis **(****FIG. 6B****).**
**FIG. 7** is a line graph demonstrating that 1-oxo-2-(2,6-dioxopiperidin-3-yl)-4-aminoisoindoline inhibits the production of the proangiogenic growth factor VEGF from sensitive NHL cells (n = 2-3).
**FIG. 8** is a line graph demonstrating The effect of 1-oxo-2-(2,6-dioxopiperidin-3-yl)-4-aminoisoindoline on Rec-1 cell growth in the presence of rhVEGF or anti-VEGF antibody (n = 2).
**FIG. 9** is a set of bar graphs demonstrating real-time RT-PCR analysis of gene expression **(****FIG. 9A****:** SPARC and **FIG. 9B****:** p21) in NHL cells treated with 1-oxo-2-(2,6-dioxopiperidin-3-yl)-4-aminoisoindoline for 24 hours.
**FIG. 10** is a bar graph demonstrating the synergistic effect of 1-oxo-2-(2,6-dioxopiperidin-3-yl)-4-aminoisoindoline and dexamethasone in the upregulation of SPARC and p21cip/kip expression in Namalwa cells at 48 hours after initiation of drug treatment.
**FIG. 11** is a time kinetic analysis of gene expression in Namalwa cells treated with 10 µM 1-oxo-2-(2,6-dioxopiperidm-3-yl)-4-aminoisoindoline for 2-48 hours.
**FIG. 12** is a pair of bar graphs demonstrating that a SPARC knockdown **(****FIG. 12A****)** impairs the antiproliferative effect of 1-oxo-2-(2,6-dioxopiperidin-3-yl)-4-aminoisoindoline on Namalwa cells **(****FIG. 12B****).**

### 5. DETAILED DESCRIPTION

Provided herein are based, in part, on the discovery that the presence and level of certain mRNAs or proteins in cell samples can be utilized as biomarkers to indicate the effectiveness or progress of a disease treatment. In particular, these mRNA or protein biomarkers can be used to predict, assess and track the effectiveness of patient treatment with various immunomodulatory compounds.

Without being limited to a particular theory, immunomodulatory compounds can mediate growth inhibition, apoptosis and inhibition of angiogenic factors in certain types of cancer such as NHL. Upon examining the expression of several cancer-related genes in several cell types before and after the treatment with an immunomodulatory compound, it was discovered that the expression levels of several cancer-related genes or proteins can be used as biomarkers for predicting and monitoring cancer treatments.

### 5.1 Definitions

As used herein, and unless otherwise specified, the terms "treat," "treating" and "treatment" refer to an action that occurs while a patient is suffering from the specified cancer, which reduces the severity of the cancer, or retards or slows the progression of the cancer.

The term "sensitivity" and "sensitive" when made in reference to treatment with an immunomodulatory compound is a relative term which refers to the degree of effectiveness of the immunomodulatory compound in lessening or decreasing the progress of a tumor or the disease being treated. For example, the term "increased sensitivity" when used in reference to treatment of a cell or tumor in connection with an immunomodulatory compound refers to an increase of, at least a 5%, or more, in the effectiveness of the tumor treatment.

As used herein, and unless otherwise specified, the term "therapeutically effective amount" of a compound is an amount sufficient to provide a therapeutic benefit in the treatment or management of a cancer, or to delay or minimize one or more symptoms associated with the presence of the cancer. A therapeutically effective amount of a compound means an amount of therapeutic agent, alone or in combination with other therapies, which provides a therapeutic benefit in the treatment or management of the cancer. The term "therapeutically effective amount" can encompass an amount that improves overall therapy, reduces or avoids symptoms or causes of cancer, or enhances the therapeutic efficacy of another therapeutic agent.

As used herein, an "effective patient tumor response" refers to any increase in the therapeutic benefit to the patient. An "effective patient tumor response" can be, for example, a 5%, 10%, 25%, 50%, or 100% decrease in the rate of progress of the tumor. An "effective patient tumor response" can be, for example, a 5%, 10%, 25%, 50%, or 100% decrease in the physical symptoms of a cancer. An "effective patient tumor response" can also be, for example, a 5%, 10%, 25%, 50%, 100%, 200%, or more increase in the general health of the patient, as measured by any suitable means, such as gene expression, cell counts, assay results, etc.

The term "likelihood" generally refers to an increase in the probability of an event. The term "likelihood" when used in reference to the effectiveness of a patient tumor response generally contemplates an increased probability that the rate of tumor progress or tumor cell growth will decrease. The term "likelihood" when used in reference to the effectiveness of a patient tumor response can also generally mean the increase of indicators, such as mRNA or protein expression, that may evidence an increase in the progress in treating the tumor.

The term "predict" generally means to determine or tell in advance. When used to "predict" the effectiveness of a cancer treatment, for example, the term "predict" can mean that the likelihood of the outcome of the cancer treatment can be determined at the outset, before the treatment has begun, or before the treatment period has progressed substantially.

The term "monitor," as used herein, generally refers to the overseeing, supervision, regulation, watching, tracking, or surveillance of an activity. For example, the term "monitoring the effectiveness of an immunomodulatory compound" refers to tracking the effectiveness in treating a cancer in a patient or in a tumor cell culture. Similarly, the "monitoring," when used in connection with patient compliance, either individually, or in a clinical trial, refers to the tracking or confirming that the patient is actually taking the immunomodulatory compound being tested as prescribed. The monitoring can be performed, for example, by following the expression of mRNA or protein biomarkers such as SPARC, cyclin D1 and p21.

An improvement in the cancer or cancer-related disease can be characterized as a complete or partial response. "Complete response" refers to an absence of clinically detectable disease with normalization of any previously abnormal radiographic studies, bone marrow, and cerebrospinal fluid (CSF) or abnormal monoclonal protein measurements. "Partial response" refers to at least about a 10%, 20%, 30%, 40%, 50%, 60%, 70%, 80%, or 90% decrease in all measurable tumor burden (i.e., the number of malignant cells present in the subject, or the measured bulk of tumor masses or the quantity of abnormal monoclonal protein) in the absence of new lesions. The term "treatment" contemplates both a complete and a partial response.

"Tumor," as used herein, refers to all neoplastic cell growth and proliferation, whether malignant or benign, and all pre-cancerous and cancerous cells and tissues. "Neoplastic," as used herein, refers to any form of dysregulated or unregulated cell growth, whether malignant or benign, resulting in abnormal tissue growth. Thus, "neoplastic cells" include malignant and benign cells having dysregulated or unregulated cell growth.

The terms "cancer" and "cancerous" refer to or describe the physiological condition in mammals that is typically characterized by unregulated cell growth. Examples of cancer include, but are not limited to, lymphoma and leukemia, and solid tumors.

As used herein the terms "polypeptide" and "protein" as used interchangeably herein, refer to a polymer of amino acids of three or more amino acids in a serial array, linked through peptide bonds. The term "polypeptide" includes proteins, protein fragments, protein analogues, oligopeptides and the like. The term polypeptide as used herein can also refer to a peptide. The amino acids making up the polypeptide may be naturally derived, or may be synthetic. Exemplary polypeptides disclosed herein include but are not limited to SPARC, cyclin D 1, p21, and the like. The polypeptide can be purified from a biological sample.

The term "antibody" is used herein in the broadest sense and covers fully assembled antibodies, antibody fragments which retain the ability to specifically bind to the antigen (*e.g*., Fab, F(ab')2, Fv, and other fragments), single chain antibodies, diabodies, antibody chimeras, hybrid antibodies, bispecific antibodies, humanized antibodies, and the like. The term "antibody" covers both polyclonal and monoclonal antibodies.

The term "expressed" or "expression" as used herein refers to the transcription from a gene to give an RNA nucleic acid molecule at least complementary in part to a region of one of the two nucleic acid strands of the gene. The term "expressed" or "expression" as used herein also refers to the translation from the RNA molecule to give a protein, a polypeptide or a portion thereof.

An mRNA that is "upregulated" is generally increased upon a given treatment or condition. An mRNA that is "downregulated" generally refers to a decrease in the level of expression of the mRNA in response to a given treatment or condition. In some situations, the mRNA level can remain unchanged upon a given treatment or condition.

An mRNA from a patient sample can be "upregulated" when treated with an immunomodulatory compound, as compared to a non-treated control. This upregulation can be, for example, an increase of about 5%, 10%, 20%, 30%, 40%, 50%, 60%, 70%, 90%, 100%, 200%, 300%, 500%, 1,000%, 5,000% or more of the comparative control mRNA level.

Alternatively, an mRNA can be "downregulated", or expressed at a lower level, in response to administration of certain immunomodulatory compounds or other agents. A downregulated mRNA can be, for example, present at a level of about 99%, 95%, 90%, 80%, 70%, 60%, 50%, 40%, 30%, 20%, 10%, 1% or less of the comparative control mRNA level.

Similarly, the level of a polypeptide or protein biomarker from a patient sample can be increased when treated with an immunomodulatory compound, as compared to a non-treated control. This increase can be about 5%, 10%, 20%, 30%, 40%, 50%, 60%, 70%, 90%, 100%, 200%, 300%, 500%, 1,000%, 5,000% or more of the comparative control protein level.

Alternatively, the level of a protein biomarker can be decreased in response to administration of certain immunomodulatory compounds or other agents. This decrease can be, for example, present at a level of about 99%, 95%, 90%, 80%, 70%, 60%, 50%, 40%, 30%, 20%, 10%, 1% or less of the comparative control protein level.

The term "immunomodulatory agent" or "immunomodulatory drug" or "immunomodulatory compound" refers to a molecule or compound, such as a small molecule or drug, an agent, a peptide, or a protein that can alter the immune system in some way. In some embodiments, the term encompasses a molecule or compound that that can inhibit LPS induced monocyte TNF-α, IL-1B, IL-12, IL-6, MIP-1α, MCP-1, GM-CSF, G-CSF, and COX-2 production.

The terms "determining", "measuring", "evaluating", "assessing" and "assaying" as used herein generally refer to any form of measurement, and include determining if an element is present or not. These terms include both quantitative and/or qualitative determinations. Assessing may be relative or absolute. "Assessing the presence of" can include determining the amount of something present, as well as determining whether it is present or absent.

The terms "nucleic acid" and "polynucleotide" are used interchangeably herein to describe a polymer of any length composed of nucleotides, e.g., deoxyribonucleotides or ribonucleotides, or compounds produced synthetically, which can hybridize with naturally occurring nucleic acids in a sequence specific manner analogous to that of two naturally occurring nucleic acids, e.g., can participate in Watson-Crick base pairing interactions. As used herein in the context of a polynucleotide sequence, the term "bases" (or "base") is synonymous with "nucleotides" (or "nucleotide"), i.e. the monomer subunit of a polynucleotide. The terms "nucleoside" and "nucleotide" are intended to include those moieties that contain not only the known purine and pyrimidine bases, but also other heterocyclic bases that have been modified. Such modifications include methylated purines or pyrimidines, acylated purines or pyrimidines, alkylated riboses or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, or are functionalized as ethers, amines, or the like. "Analogues" refer to molecules having structural features that are recognized in the literature as being mimetics, derivatives, having analogous structures, or other like terms, and include, for example, polynucleotides incorporating non-natural nucleotides, nucleotide mimetics such as 2'-modified nucleosides, peptide nucleic acids, oligomeric nucleoside phosphonates, and any polynucleotide that has added substituent groups, such as protecting groups or linking moieties.

The term "complementary" refers to specific binding between polynucleotides based on the sequences of the polynucleotides. As used herein, a first polynucleotide and a second polynucleotide are complementary if they bind to each other in a hybridization assay under stringent conditions, *e.g*. if they produce a given or detectable level of signal in a hybridization assay. Portions of polynucleotides are complementary to each other if they follow conventional base-pairing rules, *e.g.* A pairs with T (or U) and G pairs with C, although small regions (*e.g*. less than about 3 bases) of mismatch, insertion, or deleted sequence may be present.

"Sequence identity" or "identity" in the context of two nucleic acid sequences refers to the residues in the two sequences which are the same when aligned for maximum correspondence over a specified comparison window, and can take into consideration additions, deletions and substitutions.

The term "substantial identity" or "homologous" in their various grammatical forms in the context of polynucleotides generally means that a polynucleotide comprises a sequence that has a desired identity, for example, at least 60% identity, preferably at least 70% sequence identity, more preferably at least 80%, still more preferably at least 90% and even more preferably at least 95%, compared to a reference sequence. Another indication that nucleotide sequences are substantially identical is if two molecules hybridize to each other under stringent conditions.

As used herein, the term "bound" can be used herein to indicate direct or indirect attachment. In the context of chemical structures, "bound" (or "bonded") may refer to the existence of a chemical bond directly joining two moieties or indirectly joining two moieties (*e.g.* via a linking group or any other intervening portion of the molecule). The chemical bond may be a covalent bond, an ionic bond, a coordination complex, hydrogen bonding, van der Waals interactions, or hydrophobic stacking, or may exhibit characteristics of multiple types of chemical bonds. In certain instances, "bound" includes embodiments where the attachment is direct and also embodiments where the attachment is indirect.

The terms "isolated" and "purified" refer to isolation of a substance (such as mRNA or protein) such that the substance comprises a substantial portion of the sample in which it resides, i.e. greater than the substance is typically found in its natural or unisolated state. Typically, a substantial portion of the sample comprises, e.g., greater than 1%, greater than 2%, greater than 5%, greater than 10%, greater than 20%, greater than 50%, or more, usually up to about 90%-100% of the sample. For example, a sample of isolated mRNA can typically comprise at least about 1% total mRNA. Techniques for purifying polynucleotides are well known in the art and include, for example, gel electrophoresis, ion-exchange chromatography, affinity chromatography, flow sorting, and sedimentation according to density.

The term "sample" as used herein relates to a material or mixture of materials, typically, although not necessarily, in fluid form, containing one or more components of interest.

"Biological sample" as used herein refers to a sample obtained from a biological subject, including sample of biological tissue or fluid origin, obtained, reached, or collected *in vivo* or *in situ.* A biological sample also includes samples from a region of a biological subject containing precancerous or cancer cells or tissues. Such samples can be, but are riot limited to, organs, tissues, fractions and cells isolated from a mammal. Exemplary biological samples include but are not limited to cell lysate, a cell culture, a cell line, a tissue, oral tissue, gastrointestinal tissue, an organ, an organelle, a biological fluid, a blood sample, a urine sample, a skin sample, and the like. Preferred biological samples include but are not limited to whole blood, partially purified blood, PBMCs, tissue biopsies, and the like.

The term "analyte" as used herein, refers to a known or unknown component of a sample.

The term "capture agent," as used herein, refers to an agent that binds an mRNA or protein through an interaction that is sufficient to permit the agent to bind and concentrate the mRNA or protein from a homogeneous mixture.

The term "probe" as used herein, refers to a capture agent that is directed to a specific target mRNA biomarker sequence. Accordingly, each probe of a probe set has a respective target mRNA biomarker. A probe/target mRNA duplex is a structure formed by hybridizing a probe to its target mRNA biomarker.

The term "nucleic acid" or "oligonucleotide probe" refers to a nucleic acid capable of binding to a target nucleic acid of complementary sequence, such as the mRNA biomarkers provided herein, through one or more types of chemical bonds, usually through complementary base pairing, usually through hydrogen bond formation. As used herein, a probe may include natural (*e.g*., A, G, C, or T) or modified bases (7-deazaguanosine, inosine, etc.). In addition, the bases in a probe may be joined by a linkage other than a phosphodiester bond, so long as it does not interfere with hybridization. It will be understood by one of skill in the art that probes may bind target sequences lacking complete complementarity with the probe sequence depending upon the stringency of the hybridization conditions. The probes are preferably directly labeled with isotopes, for example, chromophores, lumiphores, chromogens, or indirectly labeled with biotin to which a streptavidin complex may later bind. By assaying for the presence or absence of the probe, one can detect the presence or absence of a target mRNA biomarker of interest.

The term "stringent assay conditions" refers to conditions that are compatible to produce binding pairs of nucleic acids, *e.g*., probes and target mRNAs, of sufficient complementarity to provide for the desired level of specificity in the assay while being generally incompatible to the formation of binding pairs between binding members of insufficient complementarity to provide for the desired specificity. The term stringent assay conditions generally refers to the combination of hybridization and wash conditions.

A "label" or a "detectable moiety" in reference to a nucleic acid, refers to a composition that, when linked with a nucleic acid, renders the nucleic acid detectable, for example, by spectroscopic, photochemical, biochemical, immunochemical, or chemical means. Exemplary labels include, but are not limited to, radioactive isotopes, magnetic beads, metallic beads, colloidal particles, fluorescent dyes, enzymes, biotin, digoxigenin, haptens, and the like. A "labeled nucleic acid or oligonucleotide probe" is generally one that is bound, either covalently, through a linker or a chemical bond, or noncovalently, through ionic bonds, van der Waals forces, electrostatic attractions, hydrophobic interactions, or hydrogen bonds, to a label such that the presence of the nucleic acid or probe can be detected by detecting the presence of the label bound to the nucleic acid or probe.

The terms "Polymerase chain reaction," or "PCR," as used herein generally refers to a procedure wherein small amounts of a nucleic acid, RNA and/or DNA, are amplified as described, for example, in U.S. Pat. No. 4,683,195 to Mullis. Generally, sequence information from the ends of the region of interest or beyond needs to be available, such that oligonucleotide primers can be designed; these primers will be identical or similar in sequence to opposite strands of the template to be amplified. The 5' terminal nucleotides of the two primers may coincide with the ends of the amplified material. PCR can be used to amplify specific RNA sequences, specific DNA sequences from total genomic DNA, and cDNA transcribed from total cellular RNA, bacteriophage or plasmid sequences, etc. *See generally* Mullis et al., Cold Spring Harbor Symp. Quant. Biol., 51: 263 (1987); Erlich, ed., PCR Technology, (Stockton Press, NY, 1989).

The term "cycle number" or "CT" when used herein in reference to PCR methods, refers to the PCR cycle number at which the fluorescence level passes a given set threshold level. The CT measurement can be used, for example, to approximate levels of mRNA in an original sample. The CT measurement is often used in terms of "dCT" or the "difference in the CT" score, when the CT of one nucleic acid is subtracted from the CT of another nucleic acid.

As used herein, and unless otherwise indicated, the term "optically pure" means a composition that comprises one optical isomer of a compound and is substantially free of other isomers of that compound. For example, an optically pure composition of a compound having one chiral center will be substantially free of the opposite enantiomer of the compound. An optically pure composition of a compound having two chiral centers will be substantially free of other diastereomers of the compound. A typical optically pure compound comprises greater than about 80% by weight of one enantiomer of the compound and less than about 20% by weight of other enantiomers of the compound, more preferably greater than about 90% by weight of one enantiomer of the compound and less than about 10% by weight of the other enantiomers of the compound, even more preferably greater than about 95% by weight of one enantiomer of the compound and less than about 5% by weight of the other enantiomers of the compound, more preferably greater than about 97% by weight of one enantiomer of the compound and less than about 3% by weight of the other enantiomers of the compound, and most preferably greater than about 99% by weight of one enantiomer of the compound and less than about 1% by weight of the other enantiomers of the compound.

The practice of the embodiments provided herein will employ, unless otherwise indicated, conventional techniques of molecular biology, microbiology, and immunology, which are within the skill of those working in the art. Such techniques are explained fully in the literature. Examples of particularly suitable texts for consultation include the following: Sambrook et al. (1989) Molecular Cloning; A Laboratory Manual (2d ed.); D.N Glover, ed. (1985) DNA Cloning, Volumes I and II; M.J. Gait, ed. (1984) Oligonucleotide Synthesis*;* B.D. Hames & SJ. Higgins, eds. (1984) Nucleic Acid Hybridization*;* B.D. Hames & S.J. Higgins, eds. (1984) Transcription and Translation*;* R.I. Freshney, ed. (1986) Animal Cell Culture*;* Immobilized Cells and Enzymes (IRL Press, 1986); Immunochemical Methods in Cell and Molecular Biology (Academic Press, London); Scopes (1987) Protein Purification: Principles and Practice (2d ed.; Springer Verlag, N.Y.); and D.M. Weir and C. C. Blackwell, eds. (1986) Handbook of Experimental Immunology, Volumes I-IV.

### 5.2 Non-Hodakin's Lymphoma

Malignant lymphomas are neoplastic transformations of cells that reside predominantly within lymphoid tissues. Two groups of malignant lymphomas are Hodgkin's lymphoma and non-Hodgkin's lymphoma (NHL). Both types of lymphomas infiltrate reticuloendothelial tissues. However, they differ in the neoplastic cell of origin, site of disease, presence of systemic symptoms, and response to treatment (Freedman et al., "Non-Hodgkin's Lymphomas" Chapter 134, Cancer Medicine, (an approved publication of the American Cancer Society, B.C. Decker Inc., Hamilton, Ontario, 2003).

Examples of one type of lymphoma, non-Hodgkin's lymphoma, include but are not limited to Adult T-Cell Lymphoma/Leukemia (ATLL), Anaplastic Large Cell Lymphoma (ALCL), Angiocentric Nasal T-Cell Lymphoma, Angiocentric Pulmonary B-Cell Lymphoma, Angioimmunoblastic Lymphoma, Burkitt's Lympoma (See Small Non-Cleaved Cell Lymphoma), Centrocytic Lymphoma (See Mantle Cell Lymphoma), Cutaneous B-Cell Lymphoma, Cutaneous Marginal Zone Lymphoma (MZL), Diffuse Large Cell Lymphoma (DLBCL), Diffuse Mixed Small and Large Cell Lympoma, Diffuse Small Cleaved Cell, Diffuse Small Lymphocytic Lymphoma, Enteropathy-Type T-Cell Lymphoma, Extranodal Marginal Zone B-cell lymphoma, Extranodal NK/T-Cell Lymphoma - Nasal Type, Follicular Lymphoma, Follicular Small Cleaved Cell (Grade 1), Follicular Mixed Small Cleaved and Large Cell (Grade 2), Follicular Large Cell (Grade 3), Diffuse Small Cleaved Cell, Hepatosplenic T-Cell Lymphoma, Immunoblastic Lymphoma, Intermediate Differentiation Lymphoma, Intestinal T-Cell Lymphoma, Intravascular Large B-Cell Lymphoma, Intravascular Lymphomatosis, Large Cell Immunoblastic Lymphoma, Large Cell Lymphoma (LCL), Lymphoblastic Lymphoma, Lymphomatoid Granulomatosis, MALT Lymphoma, Mantle Cell Lymphoma (MCL), Mediastinal Large B-Cell Lymphoma, Monocytoid B-cell Lymphoma, Mycosis Fungoides, Cutaneous T-Cell Lymphoma, NK-Cell Lymphoma, Nodal Marginal Zone B-cell Lymphoma, Peripheral T-Cell Lymphoma (PTL), Pleomorphic T-Cell Lymphoma, Post-Transplantation Lymphoproliferative Disorder (PTLD), Precursor B-Lymphoblastic Lymphoma, Precursor T-Cell Lymphoblastic Lymphoma, Primary Central Nervous System Lymphoma (CNS), Primary Cutaneous Anaplastic Large Cell Lymphoma/Lymphomatoid Papulosis (CD30+), Primary Effusion Lymphoma, Primary Mediastinal B-cell Lymphoma, Sezary Syndrome, Small Lymphocytic Lymphoma, Small Non-Cleaved Cell Lymphoma (SNCL), Endemic Burkitt's Lymphoma, Sporadic Burkitt's Lymphoma, Non-Burkitt's Lymphoma, Splenic Marginal Zone Lymphoma, Subcutaneous Panniculitis-Like T-Cell Lymphoma, True Histiocytic Lymphoma, Waldenstrom's Macroglobulinemia, Lymphoplasmacytic Lymphoma, and the like.

Mantle cell lymphoma (MCL) is one type of non-Hodgkin's lymphoma that represents about 6% of all B-cell non-Hodgkin's lymphomas (B-NHL) (Jaffe, et al. ed., World health organization classification of tumours. Pathology and Genetics of Tumours of Haematopoietic and Lymphoid Tissues. Lyon: IARC Press, 2001). MCL typically involves a t(11;14)(q13 ;q32) translocation. Patients with MCL often have characteristics such as a blastic morphological variant, increased tumor cell proliferation, INK4a/ARF locus deletion and p53 mutation or protein overexpression (Campo et al., (1999) Semin Hematol 36:115-127). The disease has a median patient survival of three to four years. Several of the cell lines described herein, such as Rec-1, Jeko-1, Granta-519, and JVM-2 are mantle cell lymphomas.

### 5.3 Biomarkers

Provided herein are methods relating to the use of mRNAs or proteins as biomarkers to ascertain the effectiveness of immunomodulatory agents. mRNA or protein levels can be used to determine whether a potential immunomodulatory agent is likely to be successful in cell models of disease.

A biological marker or "biomarker" is a substance whose detection indicates a particular biological state, such as, for example, the presence of cancer. In some embodiments, biomarkers can either be determined individually, or several biomarkers can be measured simultaneously.

In some embodiments, a "biomarker" indicates a change in the level of mRNA expression that may correlate with the risk or progression of a disease, or with the susceptibility of the disease to a given treatment. In some embodiments, the biomarker is a nucleic acid, such as a mRNA or cDNA.

In additional embodiments, a "biomarker" indicates a change in the level of polypeptide or protein expression that may correlate with the risk, susceptibility to treatment, or progression of a disease. In some embodiments, the biomarker can be a polypeptide or protein, such as SPARC, cyclin D1, p21, or a fragment thereof. The relative level of specific proteins can be determined by methods known in the art. For example, antibody based methods, such as an immunoblot, enzyme-linked immunosorbent assay (ELISA), or other methods can be used.

### 5.3.1 Use of SPARC mRNA or protein as a biomarker as an early indicator (or predictor) of treatment success

Based, in part, on the finding that detectable increases in SPARC mRNA expression are visible in less than 24 hours after administration of an immunomodulatory compound in sensitive cell lines but not in resistant cell lines, SPARC mRNA or protein levels may be used as a biomarker for predicting the sensitivity of a cancer cell to an immunomodulatory compound.

Thus, in some embodiments, the SPARC mRNA or protein biomarker can be used to predict the effectiveness of an immunomodulatory treatment in a patient. In an embodiment, the level of the mRNA or protein is measured in a biological sample obtained from a potential patient. An immunomodulatory compound is then administered directly to the patient. After a certain time, such as, for example, 24 hours, another sample is obtained, and the level of the SPARC mRNA or protein biomarker is compared to the level prior to administration of the compound, using, for example, RT-PCR based methods. An increased SPARC expression level after administration indicates the likelihood of effectiveness of the treatment in the patient.

Alternatively, SPARC can also be used as a biomarker for an *in vitro* assay to predict the success of an immunomodulatory treatment, by taking a sample of cancer cells from the patient, culturing them in the presence or absence of an immunomodulatory compound, and testing the cells for an increase in SPARC expression. Patients having cell samples that exhibit an increased expression of SPARC in the cell-based assay could then be treated with an immunomodulatory compound.

### 5.3.2 Monitoring progress of patient treatment using SPARC mRNA or protein expression as a biomarker

In addition to the initial prediction of the likelihood of treatment effectiveness in a patient with NHL, the progress of cancer treatment with an immunomadulatory compound can be followed using the expression of SPARC as a biomarker. Thus, in some embodiments, a method of assessing or monitoring the effectiveness of a treatment by an immunomodulatory compound in a patient is provided. A sample is obtained from the patient, and the SPARC mRNA or protein level is measured to determine whether it is present at an increased or decreased level compared to the level prior to the initiation of treatment.

NHL patients can submit the cell sample by any desired means, such as, for example, a lymph node biopsy, bone marrow biopsy, or from a circulating tumor. Samples can be taken, for example, every day, once per week, twice per month, once a month, once every two months, quarterly, or yearly, as needed to follow the effectiveness of the treatment. In an embodiment, an increase in SPARC expression after administration indicates that the treatment protocol is effective. In another embodiment, a lack of an increase in SPARC expression after administration of the immunomodulatory compound indicates that the treatment may not be effective in the particular patient, and that other treatment methods may need to be pursued. By following the SPARC mRNA or protein level, the treatment effectiveness can be monitored over time.

The mRNA or protein-based biomarkers can also be used to track and adjust individual patient treatment effectiveness. mRNA or protein-based biomarkers can be used to gather information needed to make adjustments in a patient's treatment, increasing or decreasing the dose of an agent as needed. For example, a patient receiving an immunomodulatory compound can be tested using a SPARC mRNA or protein-based biomarker to see if the dosage is becoming effective, or if a more aggressive treatment plan may be needed.

### 5.3.3 Use of cyclin D1 and p21 as prediction biomarkers

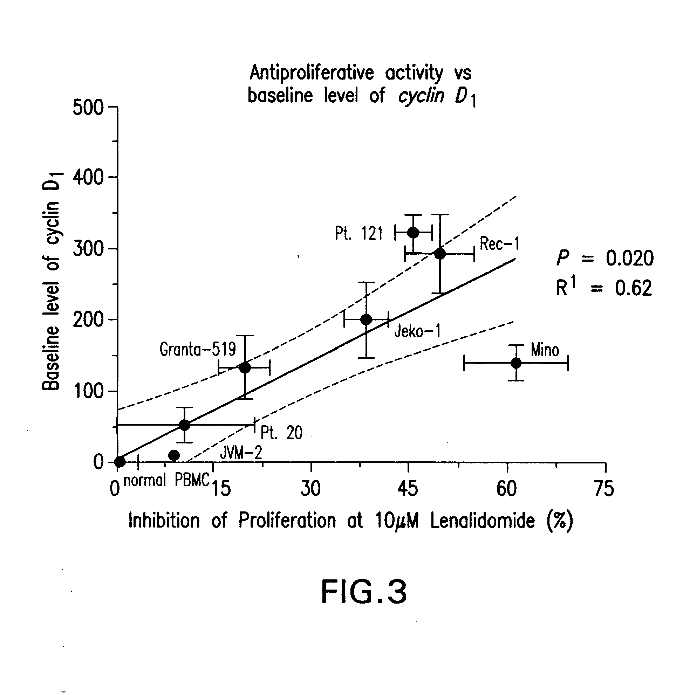

The level of cyclin D1 or p21 in the various cancer cell types allows for the prediction of likelihood of successful treatment with immunomodulatory compounds, such as 1-oxo-2-(2,6-dioxopiperidin-3-yl)-4-aminoisoindoline, by testing a biological sample from the patient and comparing the baseline levels of cyclin D1 and/or p21 gene or protein expression. Thus, the mRNAs can be used as biomarkers to predict the sensitivity to cancer treatment by administration of immunomodulatory compounds. In particular, the levels of p21 and cyclin D1 can be used to determine whether a potential immunomodulatory agent is likely to be successful in treating certain types of cancer, such as NHL (e.g., MCL). Thus, as described herein, a higher baseline level of cyclin D1 correlates to a higher likelihood of increased sensitivity to immunomodulatory compounds such as 1-oxo-2-(2,6-dioxopiperidin-3-yl)-4-ammoisoindoline (FIG. 3).

In some embodiments, the level of p21 is determined by assessing the expression of its gene, *e.g.*, mRNA. In some embodiments, the gene expression is simply measured as the PCR cycle time to reach a threshold fluorescence, or "CT."

In other embodiments, the level of p21 is determined by assessing the level of protein itself. The protein level can be measured using any methods of protein quantification well-known in the art, *e.g.*, ELISA.

In some embodiments, the expression of p21 can be used as biomarkers to monitor progress of treatment effectiveness in NHL patients that are receiving treatment with immunomodulatory compounds. Such a monitoring comprises comparing the level of p21 from a sample obtained after the treatment with that of control samples obtained before the treatment or without the treatment from another subject.

### 5.3.4 Monitoring patient compliance using p21, cyclin D1, and SPARC mRNA or protein expression as a biomarker

The p21, cyclin D1, and SPARC mRNA or protein biomarkers can additionally be used to track or perform quality control on human research trials or to monitor the patient compliance to a drug regimen by providing a means to confirm that the patient is receiving specific drug treatments. These biomarkers can be used in connection with, for example, the management of patient treatment, clinical trials, and cell-based research.

In one embodiment, the p21, and SPARC mRNA or protein-based biomarkers can be used to track patient compliance during individual treatment regimes, or during clinical trials. Thus, in some embodiments, a method for assessing patient compliance with a drug treatment protocol is provided. A biological sample is obtained from the patient, and the levels of at least one of p21, or SPARC mRNA or protein is measured and compared to that of a control untreated sample. An altered expression level of the mRNA or protein biomarker compared to that of an untreated control sample indicates compliance with the protocol.

For example, the expression of SPARC mRNA or protein can be followed at set intervals during a clinical trial to ensure that the patients included in the trial are taking the drugs as instructed. The treatment of individual patients can also be followed using the procedure. For example, when at least one of p21, or SPARC mRNA or protein is measured, an altered level of the biomarker compared to that of an untreated control indicates at least partial patient compliance with the drug treatment protocol. An altered level of the mRNA or protein biomarker that is at a similar quantity to that of a positive control indicates the likelihood of full compliance with the treatment protocol.

### 5.4 Immunomodulatory Compounds

The immunomodulatory compounds, including compounds known as "IMiDs^{®"} (Celgene Corporation), are a group of compounds that can be useful to treat several types of human diseases, including certain cancers. As provided herein, these compounds can be effective in treating NHL. In some embodiments, an immunomodulatory compound can be administered to a cell sample or to a patient, and the effectiveness of the treatment can be followed using mRNA or protein biomarkers as described herein.

As used herein and unless otherwise indicated, the term "immunomodulatory compound" can encompass certain small organic molecules that inhibit LPS induced monocyte TNF-α, IL-1ß, IL-12, IL-6, MIP-1α, MCP-1, GM-CSF, G-CSF, and COX-2 production. These compounds can be prepared synthetically, or can be obtained commercially.

Exemplary immunomodulating compounds include but are not limited to N-{[2-(2,6-dioxo(3-piperidyl)-1,3-dioxoisoindolin-4-yl]methyl}cyclopropyl-carboxamide; 3 -[2-(2,6-dioxo-piperidin-3-yl)-1,3-dioxo-2,3-dihydro-1H-isoindol-4-ylmethyl]-1,1 - dimethyl-urea; (-)-3-(3,4-Dimethoxy-phenyl)-3-(1-oxo-1,3-dihydro-isoindol-2-yl)-propionamide; (+)-3-(3,4-Dimethoxy-phenyl)-3-(1-oxo-1,3-dihydro-isoindol-2-yl)-propionamide; (-)-{2-[1-(3-ethoxy-4-methoxyphenyl)-2-methylsulfonylethyl]-4-acetylaminoisomdoline-1,3-dione}; (+)-{2-[1-(3-ethoxy-4-methoxyphenyl)-2-methylsulfonylethyl]-4-acetylaminoisoindoline-1,3-dionel; Difluoro-methoxy SelCIDs; 1-phthalimido-1-(3,4-diethoxyphenyl)ethane; 3-(3,4-dimethoxyphenyl)-3-(3,5-dimethoxyphenyl)acrylo nitrile; 1-oxo-2-(2,6-dioxopiperidin-3-yl)-4-aminoisoindoline; 1,3-dioxo-2-(2,6-dioxopiperidin-3-yl)-4-aminoisoindoline; 4-amino-2-(3-methyl-2,6-dioxo-piperidine-3-yl)-isoindole-1,3-dione; 3-(3-acetoamidophthalimido)-3-(3-ethoxy-4-methoxyphenyl]-N-hydroxypropionamide; 1-oxo-2-(2,6-dioxopiperidin-3-yl)-4-methylisoindoline; Cyclopropyl-N-{2-[(1S)-1-(3-ethoxy-4-methoxyphenyl)-2-(methylsulfonyl)ethyl]-3-oxoisoindoline-4-yl}carboxamide; Substituted 2-(3-hydroxy-2,6- dioxopiperidin-5-yl) isoindoline; N-[2-(2,6-Dioxo-piperidin-3-yl)-1,3-dioxo-2,3-dihydro-1H-isoindol-5-ylmethyl]-4-trifluoromethoxybenzamide; (S)-4-chloro-N-((2-(3-methyl-2,6-dioxopiperidin-3-yl)-1,3-dioxoisoindolin-5-yl)methyl) benzamide; Pyridine-2-carboxylic acid [2-[(3S)-3-methyl-2,6-dioxo-piperidin-3-yl]-1,3-dioxo-2,3-dihydro-1H-isoindol-5-ylmethyl]-amide; (S)-N-((2-(3-methyl-2,6-dioxopiperidin-3-yl]-1,3-dioxoisoindolin-5-yl)methyl)-4-(trifluoromethyl)benzamide; 3-(2,5-dimethyl-4-oxo-4H-quinazolin-3-yl)-piperidine-2,6-dione, and the like.

The inflammatory cytokine TNF-α, which is produced by macrophages and monocytes during acute inflammation, causes a diverse range of signaling events within cells. Without being limited by a particular theory, one of the biological effects exerted by the immunomodulatory compounds disclosed herein is the reduction of myeloid cell TNF-α production. Immunomodulatory compounds disclosed herein may enhance the degradation of TNF-α mRNA.

Further, without being limited by theory, immunomodulatory compounds disclosed herein may also be potent co-stimulators of T cells and increase cell proliferation dramatically in a dose dependent manner. Immunomodulatory compounds disclosed herein may also have a greater co-stimulatory effect on the CD8+ T cell subset than on the CD4+ T cell subset. In addition, the compounds may have antiinflammatory properties against myeloid cell responses, yet efficiently co-stimulate T cells to produce greater amounts of IL-2, IFN-γ, and to enhance T cell proliferation and CD8+ T cell cytotoxic activity. Further, without being limited by a particular theory, immunomodulatory compounds disclosed herein may be capable of acting both indirectly through cytokine activation and directly on Natural Killer ("NK") cells and Natural Killer T ("NKT") cells, and increase the NK cells' ability to produce beneficial cytokines such as, but not limited to, IFN-γ, and to enhance NK and NKT cell cytotoxic activity.

Specific examples of immunomodulatory compounds include cyano and carboxy derivatives of substituted styrenes such as those disclosed in U.S. patent no. 5,929,117; 1-oxo-2-(2,6-dioxo-3-fluoropiperidin-3yl) isoindolines and 1,3-dioxo-2-(2,6-dioxo-3-fluoropiperidine-3-yl) isoindolines such as those described in U.S. patent nos. 5,874,448 and 5,955,476; the tetra substituted 2-(2,6-dioxopiperdin-3-yl)-1-oxoisoindolines described in U.S. patent no. 5,798,368; 1-oxo and 1,3-dioxo-2-(2,6-dioxopiperidin-3-yl) isoindolines (*e.g*., 4-methyl derivatives of thalidomide), substituted 2-(2,6-dioxopiperidin-3-yl) phthalimides and substituted 2-(2,6-dioxopiperidin-3-yl)-1-oxoisoindoles including, but not limited to, those disclosed in U.S. patent nos. 5,635,517, 6,281,230, 6,316,471, 6,403,613, 6,476,052 and 6,555,554; 1-oxo and 1,3-dioxoisoindolines substituted in the 4- or 5-position of the indoline ring (*e.g.,* 4-(4-amino-1,3-dioxoisoindoline-2-yl)-4-carbamoylbutanoic acid) described in U.S. patent no. 6,380,239; isoindoline-1-one and isoindoline-1,3-dione substituted in the 2-position with 2,6-dioxo-3-hydroxypiperidin-5-yl (*e.g*., 2-(2,6-dioxo-3-hydroxy-5-fluoropiperidin-5-yl)-4-aminoisoindolin-1-one) described in U.S. patent no. 6,458,810; a class of non-polypeptide cyclic amides disclosed in U.S. patent nos. 5,698,579 and 5,877,200; and isoindole-imide compounds such as those described in U.S. patent publication no. 2003/0045552 published on March 6, 2003, U.S. patent publication no. 2003/0096841 published on May 22, 2003, and International Application No. PCT/US01/50401 (International Publication No. WO 02/059106). US patent publication no. 2006/0205787 describes 4-amino-2-(3-methyl-2,6-dioxopiperidin-3-yl)-isoindole-1,3-dione compositions. US patent publication no. 2007/0049618 describes isoindole-imide compounds. In one embodiment, immunomodulatory compounds do not include thalidomide.

Various immunomodulatory compounds disclosed herein contain one or more chiral centers, and can exist as racemic mixtures of enantiomers or mixtures of diastereomers. Thus, also provided herein is the use of stereomerically pure forms of such compounds, as well as the use of mixtures of those forms. For example, mixtures comprising equal or unequal amounts of the enantiomers of a particular immunomodulatory compounds may be used. These isomers may be asymmetrically synthesized or resolved using standard techniques such as chiral columns or chiral resolving agents. *See, e.g.,* Jacques, J., et al., Enantiomers, Racemates and Resolutions (Wiley-Interscience, New York, 1981); Wilen, S. H., et al., Tetrahedron 33:2725 (1977); Eliel, E. L., Stereochemistry of Carbon Compounds (McGraw-Hill, NY, 1962); and Wilen, S. H., Tables of Resolving Agents and Optical Resolutions p. 268 (E.L. Eliel, Ed., Univ. of Notre Dame Press, Notre Dame, IN, 1972).

Immunomodulatory compounds provided herein include, but are not limited to, 1-oxo-and 1,3 dioxo-2-(2,6-dioxopiperidin-3-yl) isoindolines substituted with amino in the benzo ring as described in U.S. Patent no. 5,635,517.

These compounds have the structure I: 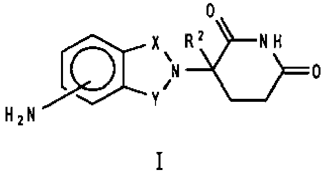 in which one of X and Y is C=O, the other of X and Y is C=O or CH₂, and R² is hydrogen or lower alkyl, in particular methyl. Specific immunomodulatory compounds include, but are not limited to: 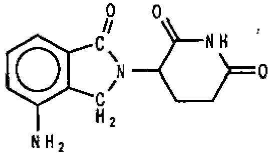 1-oxo-2-(2,6-dioxopiperidin-3-yl)-4-aminoisoindoline; 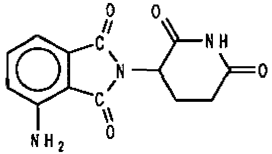 1,3-dioxo-2-(2,6-dioxopiperidin-3-yl)-4-aminoisoindoline; and 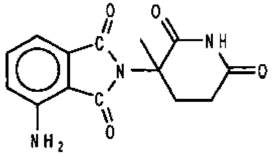 1,3-dioxo-2-(3-methyl-2,6-dioxopiperidin-3-yl)-4-aminoisoindole, and optically pure isomers thereof.

The compounds can be obtained via standard, synthetic methods (*see e.g.,* United States Patent No. 5,635,517). The compounds are also available from Celgene Corporation, Warren, NJ.

Other specific immunomodulatory compounds belong to a class of substituted 2-(2,6-dioxopiperidin-3-yl) phthalimides and substituted 2-(2,6-dioxopiperidin-3-yl)-1-oxoisoindoles, such as those described in U.S. patent nos. 6,281,230; 6,316,471; 6,335,349; and 6,476,052, and International Patent Application No. PCT/US97/13375 (International Publication No. WO 98/03502). Representative compounds are of formula: 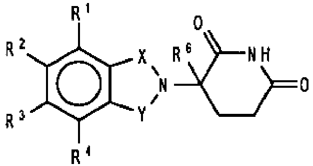 in which:
one of X and Y is C=O and the other of X and Y is C=O or CH₂;
   (i) each of R¹, R², R³, and R⁴, independently of the others, is halo, alkyl of 1 to 4 carbon atoms, or alkoxy of 1 to 4 carbon atoms or (ii) one of R¹, R², R³, and R⁴ is -NHR⁵ and the remaining of R¹, R², R³, and R⁴ are hydrogen;
   R⁵ is hydrogen or alkyl of 1 to 8 carbon atoms;
   R⁶ is hydrogen, alkyl of 1 to 8 carbon atoms, benzyl, or halo;
   provided that R⁶ is other than hydrogen if X and Y are C=O and (i) each of R¹, R², R³, and R⁴ is fluoro or (ii) one of R¹, R², R³, or R⁴ is amino.

Compounds representative of this class are of the formulas: 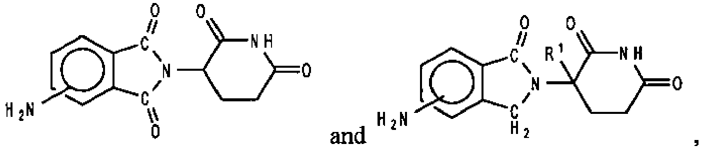 wherein R¹ is hydrogen or methyl. In a separate embodiment, provided herein is the use of enantiomerically pure forms (e.g. optically pure (R) or (S) enantiomers) of the compound.

Still other specific immunomodulatory compounds disclosed herein belong to a class of isoindole-imides disclosed in U.S. Patent No. 7,091,353, U.S. Patent Publication No. 2003/0045552, and International Application No. PCT/US01/50401 (International Publication No. WO 02/059106). Representative compounds are of formula II: 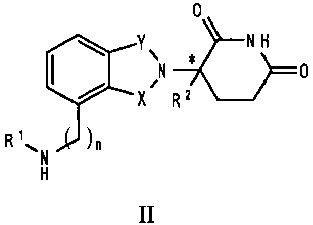 and pharmaceutically acceptable salts, hydrates, solvates, clathrates, enantiomers, diastereomers, racemates, and mixtures of stereoisomers thereof, wherein:
one of X and Y is C=O and the other is CH₂ or C=O;
R¹ is H, (C₁-C₈)alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, C(O)R³, C(S)R³, C(O)OR⁴, (C₁-C₈)alkyl-N(R⁶)2, (C₁-C₈)alkyl-OR⁵, (C₁-C₈)alkyl-C(O)OR⁵, C(O)NHR³, C(S)NHR³, C(O)NR³R^{3'}, C(S)NR³R³' or (C₁-C₈)alkyl-O(CO)R⁵;
R² is H, F, benzyl, (C₁-C₈)alkyl, (C₂-C₈)alkenyl, or (C₂-C₈)alkynyl;
R³ and R^{3'} are independently (C₁-C₈)alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, (C₀-C₈)alkyl-N(R⁶)2, (C₁-C₈)alkyl-OR⁵, (C₁-C₈)alkyl-C(O)OR⁵, (C₁-C₈)alkyl-O(CO)R⁵, or C(O)OR⁵;
R⁴ is (C₁-C₈)alkyl, (C₂-C₈)alkenyl, (C₂-C₈)alkynyl, (C₁-C₄)alkyl-OR⁵, benzyl, aryl, (C₀-C₄)alkyl-(C₁-C₆)heterocycloalkyl, or (C₀-C₄)alkyl-(C₂-C₅)heteroaryl;
R⁵ is (C₁-C₈)alkyl, (C₂-C₈)alkenyl, (C₂-C₈)alkynyl, benzyl, aryl, or (C₂-C₅)heteroaryl; each occurrence of R⁶ is independently H, (C₁-C₈)alkyl, (C₂-C₈)alkenyl, (C₂-C₈)alkynyl, benzyl, aryl, (C₂-C₅)heteroaryl, or (C₀-C₈)alkyl-C(O)O-R⁵ or the R⁶ groups can join to form a heterocycloalkyl group;
n is 0 or 1; and
* represents a chiral-carbon center.

In specific compounds of formula II, when n is 0 then R¹ is (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, C(O)R³, C(O)OR⁴, (C₁-C₈)alkyl-N(R⁶)₂, (C₁-C₈)alkyl-OR⁵, (C₁-C₈)alkyl-C(O)OR⁵, C(S)NHR³, or (C₁-C₈)alkyl-O(CO)R⁵;
R² is H or (C₁-C₈)alkyl; and
R³ is (C₁-C₈)alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, (C₅-C₈)alkyl-N(R⁶)2; (C₀-C₈)alkyl-NH-C(O)O-R⁵; (C₁-C₈)alkyl-OR⁵, (C₁-C₈)alkyl-C(O)OR⁵, (C₁-C₈)alkyl-O(CO)R⁵, or C(O)OR⁵; and the other variables have the same definitions.

In other specific compounds of formula II, R² is H or (C₁-C₄)alkyl.

In other specific compounds of formula II, R¹ is (C₁-C₈)alkyl or benzyl.

In other specific compounds of formula II, R¹ is H, (C₁-C₈)alkyl, benzyl, CH₂OCH₃, CH₂CH₂OCH₃, or

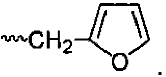

In other specific compounds of formula II, R¹ is 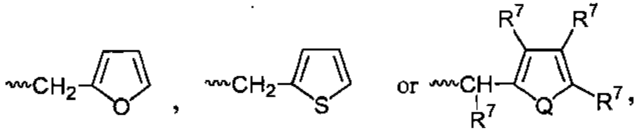 wherein Q is O or S, and each occurrence of R⁷ is independently H,(C₁-C₈)alkyl, (C₃-C₇)cycloalkyl, (C₂-C₈)alkenyl, (C₂-C₈)alkynyl, benzyl, aryl, halogen, (C₀-C₄)alkyl-(C₁-C₆)heterocycloalkyl, (C₀-C₄)alkyl-(C₂-C₅)heteroaryl, (C₀-C₈)alkyl-N(R⁶)₂, (C₁-C₈)alkyl-OR⁵, (C₁-C₈)alkyl-C(O)OR⁵, (C₁-C₈)alkyl-O(CO)R⁵, or C(O)OR⁵, or adjacent occurrences of R⁷ can be taken together to form a bicyclic alkyl or aryl ring.

In other specific compounds of formula II, R¹ is C(O)R³.

In other specific compounds of formula II, R³ is (C₀-C₄)alkyl-(C₂-C₅)heteroaryl, (C₁-C₈)alkyl, aryl, or (C₀-C₄)alkyl-OR⁵.

In other specific compounds of formula II, heteroaryl is pyridyl, furyl, or thienyl.

In other specific compounds of formula II, R¹ is C(O)OR⁴.

In other specific compounds of formula II, the H of C(O)NHC(O) can be replaced with (C₁-C₄)alkyl, aryl, or benzyl.

Further examples of the compounds in this class include, but are not limited to: [2-(2,6-dioxo-piperidin-3-yl)-1,3-dioxo-2,3-dihydro-1H-isoindol-4-ylmethyl]-amide; (2-(2,6-dioxo-piperidin-3-yl)-1,3-dioxo-2,3-dihydro-1*H*-isoindol-4-ylmethyl)-carbamic acid *tert*-butyl ester; 4-(aminomethyl)-2-(2,6-dioxo(3-piperidyl))-isoindoline-1,3-dione; *N-*(2-(2,6-dioxo-piperidin-3-yl)-1,3-dioxo-2,3-dihydro-1*H*-isoindol-4-ylmethyl)-acetamide; *N*-{(2-(2,6-dioxo(3-piperidyl)-1,3-dioxoisoindolin-4-yl)methyl}cyclopropyl-carboxamide; 2-chloro-*N*-{(2-(2,6-dioxo(3-piperidyl))-1,3-dioxoisoindolin-4-yl)methyl}acetamide; *N*-(2-(2,6-dioxo(3-piperidyl))-1,3-dioxoisoindolin-4-yl)-3-pyridylcarboxamide; 3-{1-oxo-4-(benzylamino)isoindolin-2-yl}piperidine-2,6-dione; 2-(2,6-dioxo(3-piperidyl))-4-(benzylamino)isoindoline-1,3-dione; *N*-{(2-(2,6-dioxo(3-piperidyl))-1,3-dioxoisoindolin-4-yl)methyl}propanamide; *N*-{(2-(2,6-dioxo(3-piperidyl))-1,3-dioxoisoindolin-4-yl)methyl}-3-pyridylcarboxamide; *N*-{(2-(2,6-dioxo(3-piperidyl))-1,3-dioxoisoindolin-4-yl)methyl}heptanamide; *N-*{(2-(2,6-dioxo(3-piperidyl))-1,3-dioxoisoindolin-4-yl)methyl}-2-furylcarboxamide; {N-(2-(2,6-dioxo(3-piperidyl))-1,3-dioxoisoindolin-4-yl)carbamoyl}methyl acetate; *N*-(2-(2,6-dioxo(3-piperidyl))-1,3-dioxoisoindolin-4-yl)pentanamide; *N*-(2-(2,6-dioxo(3-piperidyl))-1,3-dioxoisoindolin-4-yl)-2-thienylcarboxamide; N-{[2-(2,6-dioxo(3-piperidyl))-1,3-dioxoisoindolin-4-yl]methyl}(butylamino)carboxamide; N-{[2-(2,6-dioxo(3-piperidyl))-1,3-dioxoisoindolin-4-yl] methyl}(octylamino)carboxamide; and N-{[2-(2,6-dioxo(3-piperidyl))-1,3-dioxoisoindolin-4-yl]methyl}(benzylamino)carboxamide.

Still other specific immunomodulatory compounds disclosed herein belong to a class of isoindole-imides disclosed in U.S. Patent Application Publication Nos. US 2002/0045643, International Publication No. WO 98/54170, and United States Patent No. 6,395,754. Representative compounds are of formula III: 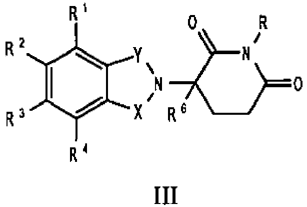 and pharmaceutically acceptable salts, hydrates, solvates, clathrates, enantiomers, diastereomers, racemates, and mixtures of stereoisomers thereof, wherein:
one of X and Y is C=O and the other is CH₂ or C=O;
R is H or CH₂OCOR';
(i) each of R¹, R², R³, or R⁴, independently of the others, is halo, alkyl of 1 to 4 carbon atoms, or alkoxy of 1 to 4 carbon atoms or (ii) one of R¹, R², R³, or R⁴ is nitro or -NHR⁵ and the remaining of R¹, R², R³, or R⁴ are hydrogen;
R⁵ is hydrogen or alkyl of 1 to 8 carbons
R⁶ hydrogen, alkyl of 1 to 8 carbon atoms, benzo, chloro, or fluoro;
R' is R⁷-CHR¹⁰-N(R⁸R⁹);
R⁷ is m-phenylene or p-phenylene or -(CnH2n)- in which n has a value of 0 to 4;
each of R⁸ and R⁹ taken independently of the other is hydrogen or alkyl of 1 to 8 carbon atoms, or R⁸ and R⁹ taken together are tetramethylene, pentamethylene, hexamethylene, or -CH₂CH₂X¹CH₂CH₂- in which X¹ is -O-, -S-, or -NH-;
R¹⁰ is hydrogen, alkyl of to 8 carbon atoms, or phenyl; and
* represents a chiral-carbon center.

Other representative compounds are of formula: 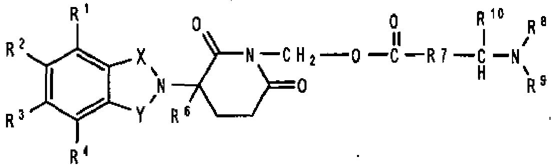 wherein:
one of X and Y is C=O and the other of X and Y is C=O or CH₂;
   (i) each of R¹, R², R³, or R⁴, independently of the others, is halo, alkyl of 1 to 4 carbon atoms, or alkoxy of 1 to 4 carbon atoms or (ii) one of R¹, R², R³, and R⁴ is -NHR⁵ and the remaining of R¹, R², R³, and R⁴ are hydrogen;
   R⁵ is hydrogen or alkyl of 1 to 8 carbon atoms;
   R⁶ is hydrogen, alkyl of 1 to 8 carbon atoms, benzo, chloro, or fluoro;
   R⁷ is m-phenylene or p-phenylene or -(CnH2n)- in which n has a value of 0 to 4;
   each of R⁸ and R⁹ taken independently of the other is hydrogen or alkyl of 1 to 8 carbon atoms, or R⁸ and R⁹ taken together are tetramethylene, pentamethylene, hexamethylene, or -CH₂CH₂X¹CH₂CH₂- in which X¹ is -O-, -S-, or -NH-; and
   R¹⁰ is hydrogen, alkyl of to 8 carbon atoms, or phenyl.

Other representative compounds are of formula: 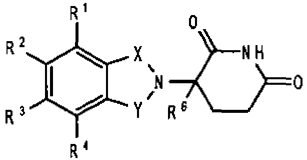 in which
one of X and Y is C=O and the other of X and Y is C=O or CH₂;
each of R¹, R², R³, and R⁴, independently of the others, is halo, alkyl of 1 to 4 carbon atoms, or alkoxy of 1 to 4 carbon atoms or (ii) one of R¹, R², R³, and R⁴ is nitro or protected amino and the remaining of R¹, R², R³, and R⁴ are hydrogen; and
R⁶ is hydrogen, alkyl of 1 to 8 carbon atoms, benzo, chloro, or fluoro.

Other representative compounds are of formula: 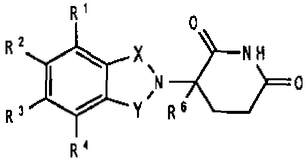 in which:
one of X and Y is C=O and the other of X and Y is C=O or CH₂;
   (i) each of R¹, R², R³, and R⁴, independently of the others, is halo, alkyl of 1 to 4 carbon atoms, or alkoxy of 1 to 4 carbon atoms or (ii) one of R¹, R², R³, and R⁴ is -NHR⁵ and the remaining of R¹, R², R³, and R⁴ are hydrogen;
   R⁵ is hydrogen, alkyl of 1 to 8 carbon atoms, or CO-R⁷-CH(R¹⁰)NR⁸R⁹ in which each of R⁷, R⁸, R⁹, and R¹⁰ is as herein defined; and
   R⁶ is alkyl of 1 to 8 carbon atoms, benzo, chloro, or fluoro.

Specific examples of the compounds are of formula: 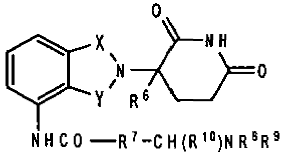 in which:
one of X and Y is C=O and the other of X and Y is C=O or CH₂;
R⁶ is hydrogen, alkyl of 1 to 8 carbon atoms, benzyl, chloro, or fluoro;
R⁷ is m-phenylene, p-phenylene or -(CnH2n)- in which n has a value of 0 to 4; each of R⁸ and R⁹ taken independently of the other is hydrogen or alkyl of 1 to 8 carbon atoms, or R⁸ and R⁹ taken together are tetramethylene, pentamethylene, hexamethylene, or -CH₂CH₂X¹CH₂CH₂- in which X¹ is -O-, -S- or -NH-; and
R¹⁰ is hydrogen, alkyl of 1 to 8 carbon atoms, or phenyl.

Other specific immunomodulatory compounds are 1-oxo-2-(2,6-dioxo-3-fluoropiperidin-3yl) isoindolines and 1,3-dioxo-2-(2,6-dioxo-3-fluoropiperidine-3-yl) isoindolines such as those described in U.S. patent nos. 5,874,448 and 5,955,476. Representative compounds are of formula: 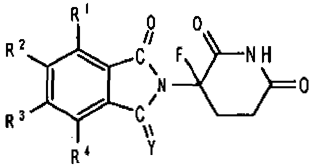 wherein:
Y is oxygen or H₂ and
each of R¹, R², R³, and R⁴, independently of the others, is hydrogen, halo, alkyl of 1 to 4 carbon atoms, alkoxy of 1 to 4 carbon atoms, or amino.

Other specific immunomodulatory compounds are the tetra substituted 2-(2,6-dioxopiperdin-3-yl)-1-oxoisoindolines described in U.S. patent no. 5,798,368. Representative compounds are of formula: 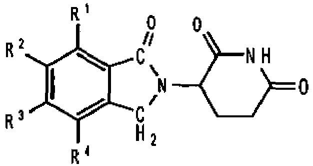 wherein each of R¹, R², R³, and R⁴, independently of the others, is halo, alkyl of 1 to 4 carbon atoms, or alkoxy of 1 to 4 carbon atoms.

Other specific immunomodulatory compounds are 1-oxo and 1,3-dioxo-2-(2,6-dioxopiperidin-3-yl) isoindolines disclosed in U.S. patent no. 6,403,613. Representative compounds are of formula: 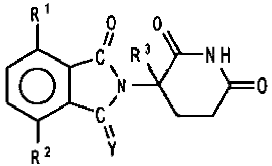 in which
Y is oxygen or H₂,
a first of R¹ and R² is halo, alkyl, alkoxy, alkylamino, dialkylamino, cyano, or carbamoyl, the second of R¹ and R², independently of the first, is hydrogen, halo, alkyl, alkoxy, alkylamino, dialkylamino, cyano, or carbamoyl, and
R³ is hydrogen, alkyl, or benzyl.

Specific examples of the compounds are of formula: 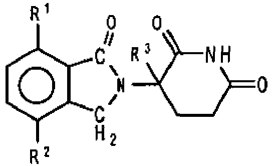 wherein
a first of R¹ and R² is halo, alkyl of from 1 to 4 carbon atoms, alkoxy of from 1 to 4 carbon atoms, dialkylamino in which each alkyl is of from 1 to 4 carbon atoms, cyano, or carbamoyl;
the second of R¹ and R², independently of the first, is hydrogen, halo, alkyl of from 1 to 4 carbon atoms, alkoxy of from 1 to 4 carbon atoms, alkylamino in which alkyl is of from 1 to 4 carbon atoms, dialkylamino in which each alkyl is of from 1 to 4 carbon atoms, cyano, or carbamoyl; and
R³ is hydrogen, alkyl of from 1 to 4 carbon atoms, or benzyl. Specific examples include, but are not limited to, 1-oxo-2-(2,6-dioxopiperidin-3-yl)-4-methylisoindoline.

Other representative compounds are of formula: 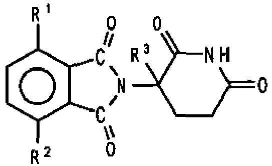 wherein:
a first of R¹ and R² is halo, alkyl of from 1 to 4 carbon atoms, alkoxy of from 1 to 4 carbon atoms, dialkylamino in which each alkyl is of from 1 to 4 carbon atoms, cyano, or carbamoyl;
the second of R¹ and R², independently of the first, is hydrogen, halo, alkyl of from 1 to 4 carbon atoms, alkoxy of from 1 to 4 carbon atoms, alkylamino in which alkyl is of from 1 to 4 carbon atoms, dialkylamino in which each alkyl is of from 1 to 4 carbon atoms, cyano, or carbamoyl; and
R³ is hydrogen, alkyl of from 1 to 4 carbon atoms, or benzyl.

Other specific immunomodulatory compounds disclosed herein are 1-oxo and 1,3-dioxoisoindolines substituted in the 4- or 5-position of the indoline ring described in U.S. Patent No. 6,380,239 and U.S. Patent No. 7,244,759. Representative compounds are of formula: 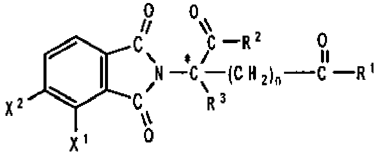 in which the carbon atom designated C* constitutes a center of chirality (when n is not zero and R¹ is not the same as R²); one of X¹ and X² is amino, nitro, alkyl of one to six carbons, or NH-Z, and the other of X¹ or X² is hydrogen; each of R¹ and R² independent of the other, is hydroxy or NH-Z; R³ is hydrogen, alkyl of one to six carbons, halo, or haloalkyl; Z is hydrogen, aryl, alkyl of one to six carbons, formyl, or acyl of one to six carbons; and n has a value of 0, 1, or 2; provided that if X¹ is amino, and n is 1 or 2, then R¹ and R² are not both hydroxy; and the salts thereof.

Further representative compounds are of formula: 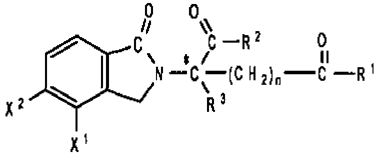 in which the carbon atom designated C* constitutes a center of chirality when n is not zero and R¹ is not R²; one of X¹ and X² is amino, nitro, alkyl of one to six carbons, or NH-Z, and the other of X¹ or X² is hydrogen; each of R¹ and R² independent of the other, is hydroxy or NH-Z; R³ is alkyl of one to six carbons, halo, or hydrogen; Z is hydrogen, aryl or an alkyl or acyl of one to six carbons; and n has a value of 0, 1, or 2.

Specific examples include, but are not limited to, 2-(4-amino-1-oxo-1,3-dihydro-isoindol-2-yl)-4-carbamoyl-butyric acid and 4-(4-amino-1-oxo-1,3-dihydro-isoindol-2-yl)-4-cabamoyl-butyric acid, which have the following structures, respectively, and pharmaceutically acceptable salts, solvates, prodrugs, and stereoisomers thereof:

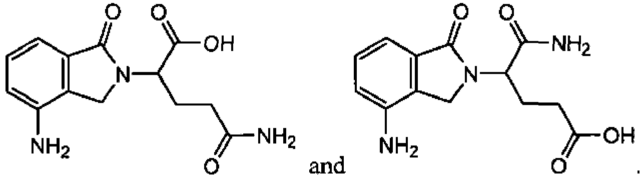

Other representative compounds are of formula: 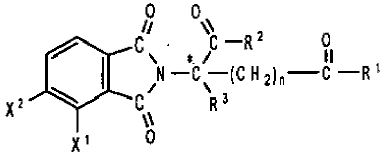 in which the carbon atom designated C* constitutes a center of chirality when n is not zero and R¹ is not R²; one of X¹ and X² is amino, nitro, alkyl of one to six carbons, or NH-Z, and the other of X¹ or X² is hydrogen; each of R¹ and R² independent of the other, is hydroxy or NH-Z; R³ is alkyl of one to six carbons, halo, or hydrogen; Z is hydrogen, aryl, or an alkyl or acyl of one to six carbons; and n has a value of 0, 1, or 2; and the salts thereof.

Specific examples include, but are not limited to, 4-carbamoyl-4-{4-[(furan-2-yl-methyl)-amino]-1,3-dioxo-1,3-dihydro-isoindol-2-yl}-butyric acid, 4-carbamoyl-2-{4-[(furan-2-yl-methyl)-amino]-1,3-dioxo-1,3-dihydro-isoindol-2-yl}-butyric acid, 2-{4-[(furan-2-yl-methyl)-amino]-1,3-dioxo-1,3-dihydro-isoindol-2-yl}-4-phenylcarbamoyl-butyric acid, and 2-{4-[(furan-2-yl-methyl)-ammo]-1,3-dioxo-1,3-dihydro-isoindol-2-yl}-pentanedioic acid, which have the following structures, respectively, and pharmaceutically acceptablesalts, solvate, prodrugs, and stereoisomers thereof:

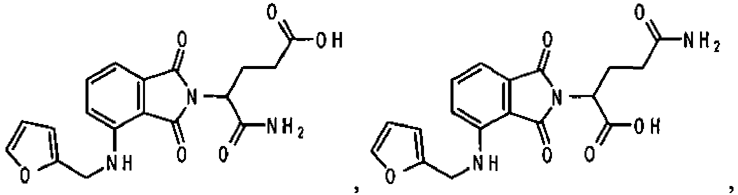

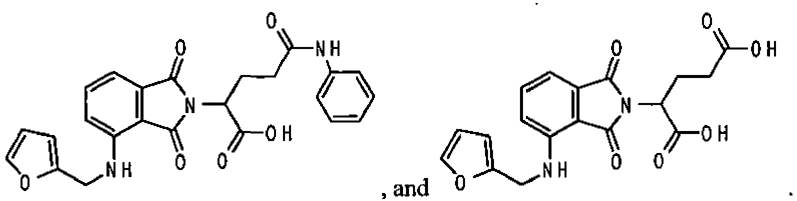

Other specific examples of the compounds are of formula: 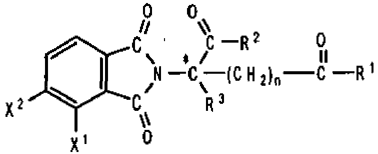 wherein:
one of X¹ and X² is nitro, or NH-Z, and the other of X¹ or X² is hydrogen;
each of R¹ and R², independent of the other, is hydroxy or NH-Z;
R³ is alkyl of one to six carbons, halo, or hydrogen;
Z is hydrogen, phenyl, an acyl of one to six carbons, or an alkyl of one to six carbons; and
n has a value of 0, 1, or 2; and
if -COR² and -(CH₂)*ₙ*COR¹ are different, the carbon atom designated C* constitutes a center of chirality.

Other representative compounds are of formula: 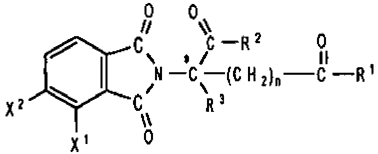 wherein:
one of X¹ and X² is alkyl of one to six carbons;
each of R¹ and R², independent of the other, is hydroxy or NH-Z;
R³ is alkyl of one to six carbons, halo, or hydrogen;
Z is hydrogen, phenyl, an acyl of one to six carbons, or an alkyl of one to six carbons; and
n has a value of 0, 1, or 2; and
if -COR² and -(CH₂)*ₙ*COR¹ are different, the carbon atom designated C* constitutes a center of chirality.

Still other specific immunomodulatory compounds are isoindoline-1-one and isoindoiine-1,3-dione substituted in the 2-position with 2,6-dioxo-3-hydroxypiperidin-5-yl described in U.S. patent no. 6,458,810. Representative compounds are of formula: 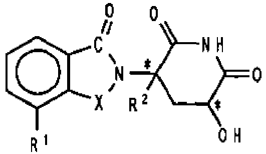 wherein:
the carbon atoms designated constitute centers of chirality;
X is -C(O)- or -CH₂-;
R¹ is alkyl of 1 to 8 carbon atoms or -NHR³;
R² is hydrogen, alkyl of 1 to 8 carbon atoms, or halogen; and
R³ is hydrogen,
alkyl of 1 to 8 carbon atoms, unsubstituted or substituted with alkoxy of 1 to 8 carbon atoms, halo, amino, or alkylamino of 1 to 4 carbon atoms,
cycloalkyl of 3 to 18 carbon atoms,
phenyl, unsubstituted or substituted with alkyl of 1 to 8 carbon atoms, alkoxy of 1 to 8 carbon atoms, halo, amino, or alkylamino of 1 to 4 carbon atoms,
benzyl, unsubstituted or substituted with alkyl of 1 to 8 carbon atoms, alkoxy of 1 to 8 carbon atoms, halo, amino, or alkylamino of 1 to 4 carbon atoms, or -COR⁴ in which
R⁴ is hydrogen,
alkyl of 1 to 8 carbon atoms, unsubstituted or substituted with alkoxy of 1 to 8 carbon atoms, halo, amino, or alkylamino of 1 to 4 carbon atoms,
cycloalkyl of 3 to 18 carbon atoms,
phenyl, unsubstituted or substituted with alkyl of 1 to 8 carbon atoms, alkoxy of 1 to 8 carbon atoms, halo, amino, or alkylamino of 1 to 4 carbon atoms, or
benzyl, unsubstituted or substituted with alkyl of 1 to 8 carbon atoms, alkoxy of 1 to 8 carbon atoms, halo, amino, or alkylamino of 1 to 4 carbon atoms.

All of the compounds described can either be commercially purchased or prepared according to the methods described in the patents or patent publications disclosed herein. Further, optically pure compounds can be asymmetrically synthesized or resolved using known resolving agents or chiral columns as well as other standard synthetic organic chemistry techniques. Additional information on immunomodulatory compounds, their preparation, and use can be found, for example, in U.S. Patent Application Publication Nos. US20060188475, US20060205787, and US20070049618.

The compounds may be small organic molecules having a molecular weight less than about 1,000 g/mol, and are not proteins, peptides, oligonucleotides, oligosaccharides or other macromolecules.

It should be noted that if there is a discrepancy between a depicted structure and a name given that structure, the depicted structure is to be accorded more weight. In addition, if the stereochemistry of a structure or a portion of a structure is not indicated with, for example, bold or dashed lines, the structure or portion of the structure is to be interpreted as encompassing all stereoisomers of it.

### 5.4.1 Methods of Administration of Immunomodulatory Compounds

Any route of administration of an immodulatory compound may be used. For example, an immunomodulatory compound can be administered by oral, parenteral, intravenous, transdermal, intramuscular, rectal, sublingual, mucosal, nasal, or other means. In addition, an immunomodulatory compounds can be administered in a form of pharmaceutical composition and/or unit dosage form. Suitable dosage forms include, but are not limited to, capsules, tablets (including rapid dissolving and delayed release tablets), powder, syrups, oral suspensions and solutions for parenteral administration. Suitable administration methods for the immunomodulatory compounds, as well as suitable dosage forms and pharmaceutical compositions, can be found in U.S. Patent Application Publication Nos. US20060188475, US20060205787, and US20070049618.

The specific amount of the agent will depend on the specific agent used, the type of disease or disorder being treated or managed, and the amount(s) of an immunomodulatory compound provided herein and any optional additional agents concurrently administered to the patient. Typical dosage forms comprise an immunomodulatory compound or a pharmaceutically acceptable salt, solvate, stereoisomer, or prodrug thereof in an amount of from about 0.001 to about 150 mg. In particular, dosage forms comprise an immunomodulatory compound or a pharmaceutically acceptable salt, solvate, stereoisomer, or prodrug thereof in an amount of about 0.001, 0.01, 0.1, 1, 2, 5, 7.5, 10, 12.5, 15, 17.5, 20,25, 50,100,150 or 200 mg. In a particular embodiment, a dosage form comprises 4-(amino)-2-(2,6-dioxo(3-piperidyl))-isoindoline-1,3-dione in an amount of about 0.001, 0.01, 0.1, 1, 2, 5, 10, 25 or 50 mg.

Pharmaceutical compositions provided herein can also contain one of more pharmaceutically acceptable excipients. *See, e.g.,* Rowe et al., Handbook of Pharmaceutical Excipients, 4th Ed. (2003).

In some embodiments, an immunomodulatory compound is administered to a subject about 3 months, 30 days, 20 days, 15 days, 12 days, 10 days, 7 days, 5 days, 3 days, 1 day, 12 hours, or 5 hours prior to testing for mRNA or protein biomarker levels. In other embodiments, an immunomodulatory compound is administered from about 3 months to about 30 days, 30 days to about 5 hours, from about 20 days to about 5 hours, from about 15 days to about 12 hours, from about 12 days to about 5 hours, from about 10 days to about 12 hours, from about 7 days to about 12 hours, from about 5 days to about 12 hours, from about 5 days to about 1 day, from about 3 days to about 12 hours, or from about 3 days to about 1 day prior to testing for mRNA or protein biomarker levels.

In some embodiments, provided herein is mRNA or protein biomarker-based monitoring upon administration of racemic mixture, optically pure (R)-isomer, or optically pure (S)-isomer of 4-(amino)-2-(2,6-dioxo(3-piperidyl))-isoindoline-1,3-dione. In one specific embodiment, the racemic 4-(amino)-2-(2,6-dioxo(3-piperidyl))-isoindoline-1,3-dione is administered at an amount of 1, 2, 5, 10, or 25 mg per day. As (S)-isomer of 4-(amino)-2-(2,6-dioxo(3-piperidyl))-isoindoline-1,3-dione is reported to have a higher potency than the racemic mixture, a lower dose can be given when (S)-isomer is used. For example, (S)- 4-(amino)-2-(2,6-dioxo(3-piperidyl))-isoindoline-1,3-dione can be administered at an amount of 0.01, 0.1, 1, 2.5, 5, or 10 mg per day. The (R)-isomer of 4-(amino)-2-(2,6-dioxo(3-piperidyl))-isoindoline-1,3-dione can be administered at an amount comparable to the racemic mixture.

As disclosed herein, a dosage form comprises 3-(4-amino-1-oxo-1,3-dihydro-isoindol-2-yl)-piperidine-2,6-dione in an amount of about 0.001, 0.01, 0.1, 1, 5, 10, 25 or 50 mg. Also provided herein is the use of racemic mixture, (S)-isomer, and (R)-isomer of 3-(4-amino-1-oxo-1,3-dihydro-isoindol-2-yl)-piperidine-2,6-dione. Typically, racemic 3-(4-amino-1-oxo-1,3-dihydro-isoindol-2-yl)-piperidine-2,6-dione can be administered at an amount of 1, 5, 10, 15, 25, or 50 mg per day. Optical isomers also can be administered at an amount comparable to racemic mixture. Doses can be adjusted depending on the type of disease or disorder being treated, prevented or managed, and the amount of an immunomodulatory compound and any optional additional agents concurrently administered to the patient, which are all within the skill of the art.

### 5.5 Methods of Detecting mRNA or Protein Levels in a Sample

Any suitable method of detecting differences the levels of mRNA or protein biomarkers SPARC, p21 can be used. In some embodiments, the biomarker to be detected is an mRNA molecule. In other embodiments, the method of measuring gene or protein expression can involve methods such as cDNA hybridization, flow cytometry, immunofluorescence, immunoblots, ELISAs or microspotted-antibody immunofluorescence assays, an antibody-based dipstick assay, cytometric bead arrays, or other common mRNA or protein detecting methods.

### 5.5.1 Methods of Detecting mRNA Levels in a Sample

Several methods of detecting or quantitating mRNA levels are known in the art. Exemplary methods include but are not limited to northern blots, ribonuclease protection assays, PCR-based methods, and the like. When the biomarker is an mRNA molecule, the mRNA sequence, e.g., SPARC, cyclin D1, p21 mRNA, or a fragment thereof, can be used to prepare a probe that is at least partially complementary. The probe can then be used to detect the mRNA sequence in a sample, using any suitable assay, such as PCR-based methods, Northern blotting, a dipstick assay, and the like.

In other embodiments, a nucleic acid assay for testing for immunomodulatory activity in a biological sample can be prepared. An assay typically contains a solid support and at least one nucleic acid contacting the support, where the nucleic acid corresponds to at least a portion of an mRNA that has altered expression during an immunomodulatory treatment in a patient, such as SPARC, cyclin D1, or p21 mRNA. The assay can also have a means for detecting the altered expression of the mRNA in the sample.

The assay method can be varied depending on the type of mRNA information desired. Exemplary methods include but are not limited to Northern blots and PCR-based methods *(e.g.,* qRT-PCR). Methods such as qRT-PCR can also accurately quantitate the amount of the mRNA in a sample.

Any suitable assay platform can be used to determine the presence of the mRNA in a sample. For example, an assay may be in the form of a dipstick, a membrane, a chip, a disk, a test strip, a filter, a microsphere, a slide, a multiwell plate, or an optical fiber. An assay system may have a solid support on which a nucleic acid corresponding to the mRNA is attached. The solid support may comprise, for example, a plastic, silicon, a metal, a resin, glass, a membrane, a particle, a precipitate, a gel, a polymer, a sheet, a sphere, a polysaccharide, a capillary, a film a plate, or a slide. The assay components can be prepared and packaged together as a kit for detecting an mRNA.

The nucleic acid can be labeled, if desired, to make a population of labeled mRNAs. In general, a sample can be labeled using methods that are well known in the art (e.g., using DNA ligase, terminal transferase, or by labeling the RNA backbone, etc.; *see, e.g.,* Ausubel, et al., Short Protocols in Molecular Biology, 3rd ed., Wiley & Sons 1995 and Sambrook et al., Molecular Cloning: A Laboratory Manual, Third Edition, 2001 Cold Spring Harbor, N.Y.). In some embodiments, the sample is labeled with fluorescent label. Exemplary fluorescent dyes include but are not limited to xanthene dyes, fluorescein dyes, rhodamine dyes, fluorescein isothiocyanate (FITC), 6 carboxyfluorescein (FAM), 6 carboxy-2',4',7',4,7-hexachlorofluorescein (HEX), 6 carboxy 4', 5'dichloro 2', 7' dimethoxyfluorescein (JOE or J), N,N,N',N' tetramethyl 6 carboxyrhodamine (TAMRA or T), 6 carboxy X rhodamine (ROX or R), 5 carboxyrhodamine 6G (R6G5 or G5), 6 carboxyrhodamine 6G (R6G6 or G6), and rhodamine 110; cyanine dyes, e.g. Cy3, Cy5 and Cy7 dyes; Alexa dyes, e.g. Alexa-fluor-555; coumarin, Diethylaminocoumarin, umbelliferone; benzimide dyes, *e.g.* Hoechst 33258; phenanthridine dyes, e.g. Texas Red; ethidium dyes; acridine dyes; carbazole dyes; phenoxazine dyes; porphyrin dyes; polymethine dyes, BODIPY dyes, quinoline dyes, Pyrene, Fluorescein Chlorotriazinyl, R110, Eosin, JOE, R6G, Tetramethylrhodamine, Lissamine, ROX, Napthofluorescein, and the like.

In some embodiments, the mRNA sequences comprise at least one mRNA selected from the group consisting of SPARC mRNA, p21 mRNA, or a fragment thereof. The nucleic acids may be present in specific, addressable locations on a solid support; each corresponding to at least a portion of mRNA sequences that are differentially expressed upon treatment of an immunomodulatory compound in a cell or a patient.

A typical mRNA assay method can contain the steps of 1) obtaining surface-bound subject probes; 2) hybridization of a population of mRNAs to the surface-bound probes under conditions sufficient to provide for specific binding (3) post-hybridization washes to remove nucleic acids not bound in the hybridization; and (4) detection of the hybridized mRNAs. The reagents used in each of these steps and their conditions for use may vary depending on the particular application.

Hybridization can be carried out under suitable hybridization conditions, which may vary in stringency as desired. Typical conditions are sufficient to produce probe/target complexes on a solid surface between complementary binding members, i.e., between surface-bound subject probes and complementary mRNAs in a sample. In certain embodiments, stringent hybridization conditions may be employed.

Hybridization is typically performed under stringent hybridization conditions. Standard hybridization techniques (e.g. under conditions sufficient to provide for specific binding of target mRNAs in the sample to the probes) are described in Kallioniemi et al., Science 258:818-821 (1992) and WO 93/18186. Several guides to general techniques are available, *e.g.,* Tijssen, Hybridization with Nucleic Acid Probes, Parts I and II (Elsevier, Amsterdam 1993). For descriptions of techniques suitable for *in situ* hybridizations, see Gall et al. Meth. Enzymol., 21:470-480 (1981); and Angerer et al. in Genetic Engineering: Principles and Methods (Setlow and Hollaender, Eds.) Vol 7, pgs 43-65 (Plenum Press, New York 1985). Selection of appropriate conditions, including temperature, salt concentration, polynucleotide concentration, hybridization time, stringency of washing conditions, and the like will depend on experimental design, including source of sample, identity of capture agents, degree of complementarity expected, etc., and may be determined as a matter of routine experimentation for those of ordinary skill in the art.

Those of ordinary skill will readily recognize that alternative but comparable hybridization and wash conditions can be utilized to provide conditions of similar stringency.

After the mRNA hybridization procedure, the surface bound polynucleotides are typically washed to remove unbound nucleic acids. Washing may be performed using any convenient washing protocol, where the washing conditions are typically stringent, as described above. The hybridization of the target mRNAs to the probes is then detected using standard techniques.

### 5.5.2 PCR-based methods of detecting mRNA biomarkers

Other methods, such as PCR-based methods, can also be used to follow the expression of the SPARC, or p21 biomarkers. Examples of PCR methods can be found in the literature. Examples of PCR assays can be found in U.S. Patent No. 6,927,024. Examples of RT-PCR methods can be found in U.S. Patent No. 7,122,799. A method of fluorescent in situ PCR is described in U.S. Patent No. 7,186,507.

In some embodiments, Real-Time Reverse Transcription-PCR (qRT-PCR) can be used for both the detection and quantification of RNA targets (Bustin, et al., 2005, Clin. Sci., 109:365-379). Quantitative results obtained by qRT-PCR are generally more informative than qualitative data. Thus, in some embodiments, qRT-PCR-based assays can be useful to measure mRNA levels during cell-based assays. The qRT-PCR method is also useful to monitor patient therapy. Examples of qRT-PCR-based methods can be found, for example, in U.S. Patent No. 7,101,663.

In contrast to regular reverse transcriptase-PCR and analysis by agarose gels, real-time PCR gives quantitative results. An additional advantage of real-time PCR is the relative ease and convenience of use. Instruments for real-time PCR, such as the Applied Biosystems 7500, are available commercially, as are the reagents, such as TaqMan Sequence Detection chemistry. For example, TaqMan^{®} Gene Expression Assays can be used, following the manufacturer's instructions. These kits are pre-formulated gene expression assays for rapid, reliable detection and quantification of human, mouse and rat mRNA transcripts. An exemplary PCR program, for example, is 50°C for 2 minutes, 95°C for 10 minutes, 40 cycles of 95°C for 15 seconds, then 60°C for 1 minute.

To determine the cycle number at which the fluorescence signal associated with a particular amplicon accumulation crosses the threshold (referred to as the CT), the data can be analyzed, for example, using a 7500 Real-Time PCR System Sequence Detection software v1.3 using the comparative CT relative quantification calculation method. Using this method, the output is expressed as a fold-change of expression levels. In some embodiments, the threshold level can be selected to be automatically determined by the software. In some embodiments, the threshold level is set to be above the baseline but sufficiently low to be within the exponential growth region of an amplification curve.

### 5.5.3 Methods of detecting polypeptide or protein biomarkers

When the biomarker is a protein, such as SPARC, Cyclin D1, or p21 protein, several protein detection and quantitation methods can be used to measure the presence of the biomarker. Any suitable protein quantitation method can be used. In some embodiments, antibody-based methods are used. Exemplary methods that can be used include but are not limited to immunoblotting (western blot), enzyme-linked immunosorbent assay (ELISA), immunohistochemistry, flow cytometry, cytometric bead array, mass spectroscopy, and the like. Several types of ELISA are commonly used, including direct ELISA, indirect ELISA, and sandwich ELISA.

### 5.6 Biological Samples

Any suitable sample can be used to assess the mRNA or protein biomarkers provided herein. In some embodiments, the biological sample is whole blood, partially purified blood, a PBMC, a tissue biopsy, an RNA or protein extract, a cell extract, a cell lysate, a cell, a cell culture, a cell line, a tissue, an oral tissue, a gastrointestinal tissue, an organ, an organelle, a biological fluid, a blood sample, a urine sample, a skin sample, a plurality of samples from a clinical trial, or the like. In an embodiment, the sample is a lymph node biopsy, a bone marrow biopsy, or a sample of peripheral blood tumor cells. The sample can be a crude sample, or can be purified to various degrees prior to storage, processing, or measurement.

Samples for mRNA or protein assessment can be taken during any desired intervals. For example, samples can be taken hourly, twice per day, daily, weekly, monthly, every other month, yearly, or the like. The sample can be tested immediately, or can be stored for later testing.

The samples can be purified prior to testing. In some embodiments, the mRNA or protein can be isolated from the remaining cell contents prior to testing. Control samples can be taken from various sources. In some embodiments, control samples are taken from the patient prior to treatment. A cell-based assay can utilize a control cell culture, for example, that has not been treated with the test compound.

### 5.7 Screening for Effective Immunomodulatory Compounds using mRNA or Protein Biomarkers

In some embodiments, a method of screening for effective immunomodulatory compounds for treating several types of NHL can be obtained using the methods provided herein. For example, an NHL cell type is chosen and cultured. Baseline SPARC mRNA or protein is measured. The cell (or cells) are then contacted with a drug candidate, or a plurality of drug candidates. After an incubation period to allow for gene expression to occur, the level of SPARC mRNA or protein is measured and compared to that of a similar untreated cell. The mRNA or protein levels are analyzed to determine whether the treated sample exhibits increased SPARC expression. Drug candidates that exhibit a pattern of increased SPARC expression can then be chosen for further studies to elucidate the activity of the candidate compound.

### 5.8 Kits for Detecting mRNA Biomarkers

In some embodiments, a kit for detecting the SPARC, and/or p21 mRNA biomarkers can be prepared. The kits can include, for example, a probe or probe set comprising oligonucleotides that can bind to the mRNA biomarker(s) of interest for a given disease, compound, or other parameter. Washing solutions, reagents for performing a hybridization assay, mRNA isolation or purification means, detection means, as well as positive and negative controls can also be included. The kit can also include instructions for using the components of the kit. The kit can be tailored for in-home use, clinical use, or research use.

### 5.9 Kits for Detecting Polypeptide or Protein Biomarkers

In some embodiments, a kit for detecting the SPARC, and/or p21 protein levels can be prepared. The kits can include, for example, a dipstick coated with an antibody that recognises the protein, washing solutions, reagents for performing the assay, protein isolation or purification means, detection means, as well as positive and negative controls. The kit can also include instructions for using the components of the kit. The kit can be tailored for in-home use, clinical use, or research use.

### 6. EXAMPLES

The examples below are carried out using standard techniques, which are well known and routine to those of skill in the art, except where otherwise described in detail. The examples are intended to be merely illustrative.

As detailed below, the effect of the administration of an immunomodulatory compound on several types of NHL cells was determined after 1-3 days using 3H-thymidine incorporation, microbead array technology and real time PCR.

### 6.1 Methods

*Cell proliferation assay:* NHL cell proliferation was assessed by 3H-thymidine incorporation assay. Briefly, cells were cultured in 96 well cell culture plates in complete RPMI-1640 medium in the presence and absence of drugs. Following incubation at 37°C for 3 days, 1 µCi 3H-thymidine was added to each well for the last 5 hours of incubation. The 3H incorporation of each well was then measured.

*Flow cytometric assay:* Cells were harvested following treatment with the test drugs and stained with propidium iodide (PI) and annexin-V-FITC. Cell cycle analysis was carried out on BD FACScanto and analyzed with the ModFit program.

*Luminex assay for growth factors:* Cells were treated with the drug for 3 days in 96 well plates. Cell culture supernatants were then collected for measurement of VEGF using Luminex/xMAP® technology.

*Real-time RT-PCR analysis:* After the cell treatment, total RNA was purified. Real-time PCR was performed with 100 ng of total RNA, using an Applied Biosystems 7500 instrument (Applied Biosystems, Foster City, CA) using TaqMan Sequence Detection chemistry which uses a fluorogenic probe (FAM) to enable the detection of a specific PCR product as it accumulates during PCR. Samples were prepared in triplicate in 50 µl reaction volumes. The 50 µl reactions consisted of 25 µl 2x TaqMan PCR master mix, 2.5 µl of 20x gene expression assay, 10 µl of RNA (500 ng) and 12.5 µl water. Glyceraldehyde-3-phosphate dehydrogenase (GAPDH) was used as the endogenous control to ensure equal RNA amounts in each sample.

TaqMan® Gene Expression Assays were pre-formulated gene expression assays for rapid, reliable detection and quantification of human, mouse and rat mRNA transcripts. The PCR program used was: 50°C for 2 minutes, 95°C for 10 minutes, 40 cycles of 95°C for 15 seconds, 60°C for 1 minute. Data was analyzed using 7500 Real-Time PCR System Sequence Detection software v1.3 using the comparative CT relative quantification calculation method. The output was expressed as a fold-change of expression levels. The threshold level was selected to be automatically determined by the software and was set to be above the baseline but sufficiently low to be within the exponential growth region of an amplification curve. The cycle number at which the fluorescence signal associated with a particular amplicon accumulation crosses the threshold is referred to as the CT.

*Small interfering RNA transfection:* Cells were transfected with SPARC or cyclophilin siGENME SMARTpool (Dharmacon, Lafayette, Co) at final concentration of 200 nM using DharmaFECT 2 transfection reagents following the manufacturer's protocol. After 24 hours of transfection, cells were then treated with the drug for 2 days.

### 6.2 Effect of 1,3-dioxo-2-(2,6-dioxopiperidin-3-yl)-4-aminoisoindoline on VEGF, p21, p53, and mRNA Expression in Rec-1 cells

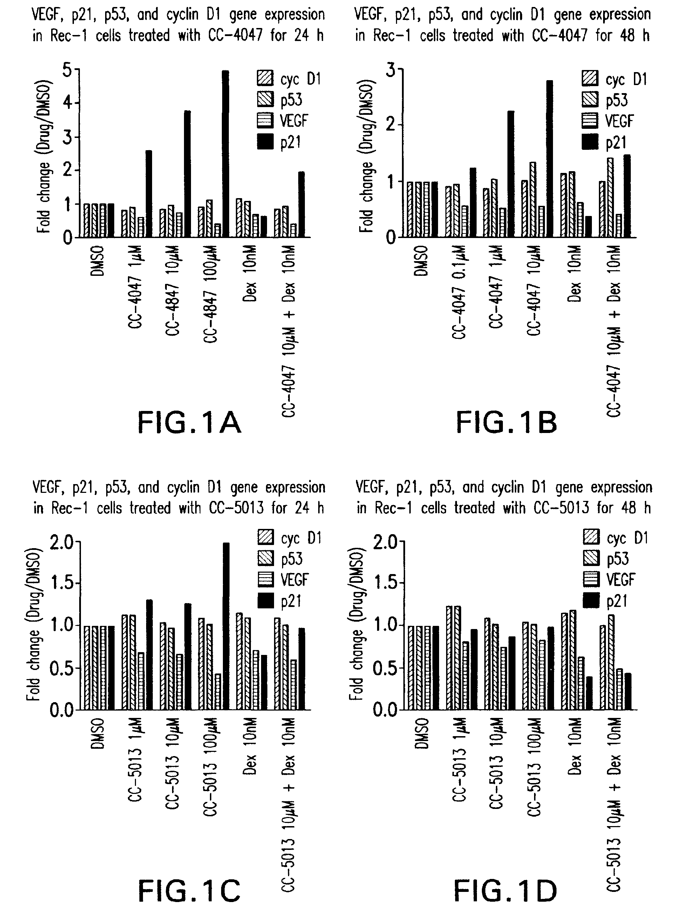

The effect of 1,3-dioxo-2-(2,5-dioxopiperidin-3-yl)-4-aminoisoindoline or 1-oxo-2-(2,6-dioxopiperidin-3-yl)-4-aminoisoindoline, either alone or in combination with Dexamethasone (10 nM) on the expression of several important genes involved in the regulation of NHL cell proliferation and survival was determined using Rec-1 cells (FIG. 1). The immunomodulatory compounds 1,3-dioxo-2-(2,6-dioxopiperidin-3-yl)-4-aminoisoindoline or 1-oxo-2-(2,6-dioxopiperidin-3-yl)-4-aminoisoindoline were added to the Rec-1 cells at a final concentration of 1, 10, or 100 µM. After 24 or 48 hours of incubation with 1,3-dioxo-2-(2,6-dioxopiperidin-3-yl)-4-aminoisoindoline or 1-oxo-2-(2,6-dioxopiperidin-3-yl)-4-aminoisoindoline, gene expression of VEGF, p21, p53, and cyclin D1 was measured using real-time PCR. A decrease in the VEGF mRNA level and an increase in the level of p21 mRNA was observed in the Rec-1 cells.

### 6.3 Effect of 1-oxo-2-(2,6-dioxopiperidin-3-yl)-4-aminoisoindoline Combined with Dexamethasone on Cell Viability in Jeko-1 Cells

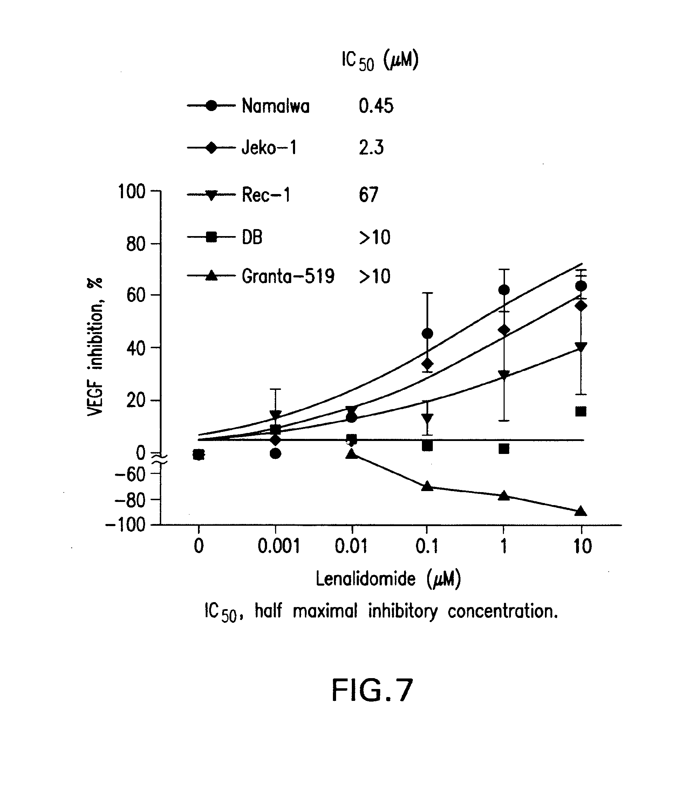

The effect of the combination of 1-oxo-2-(2,6-dioxopiperidin-3-yl)-4-aminoisoindoline and dexamethasone in Jeko-1 cells was examined. The results (FIG. 7) show that there is a marked synergy between dexamethasone and 1-oxo-2-(2,6-dioxopiperidin-3-yl)-4-aminoisoindoline in the inhibition of NHL cell growth and survival. The dexamethasone treatment for 3 days barely induced cell cycle arrest in G0/G1 phase and apoptosis. However, addition of 1-oxo-2-(2,6-dioxopiperidin-3-yl)-4-aminoisoindoline markedly enhanced the anti-proliferative effect of dexamethasone (FIG. 7).

### 6.4 Effect of the Administration of Various Immunomodulatary Agents on mRNA Level of SPARC, p21, and Activin A in Jeko-1 Cells

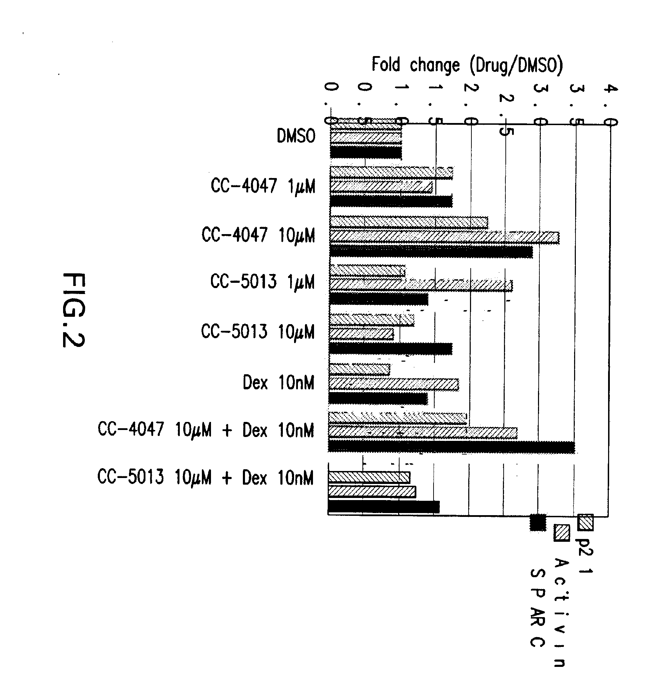

Jeko-1 cells were treated with immunomodulatory compounds, with or without dexamethasone (10 nM) for 24 hours. Total RNA was then isolated from the treated cells, and the samples were subjected to real-time RT-PCR analysis to determine gene transcription at the mRNA level. The immunomodulatory compound 1,3-dioxo-2-(2,6-dioxopiperidin-3-yl)-4-aminoisoindoline (at 1-10 µM) was found to up-regulate the mRNA levels of p21, activin A and SPARC (FIG. 2). 1,3-dioxo-2-(2,6-dioxopiperidin-3-yl)-4-aminoisoindoline (at 10 µM) enhanced mRNA levels of these genes up to about 3-fold. 1-oxo-2-(2,6-dioxopiperidin-3-yl)-4-aminoisoindoline (1 µM) enhanced activin A mRNA level up to 2.6-fold, and SPARC was enhanced at 10 µM. 1,3-dioxo-2-(2,6-dioxopiperidin-3-yl)-4-aminoisoindoline had a clear enhancing effect on p21.

### 6.5 Antiproliferative Properties of Immunomodulatary Compounds in Certain NHL Cell Lines

To better understand how the effectiveness of immunomodulatory compound administration varies among different cancer cell types, the immunomodulatory compound 1-oxo-2-(2,6-dioxopiperidin-3-yl)-4-aminoisoindoline was tested *in vitro* for its anti-proliferative effect in several NHL cell lines after 1-3 days of treatment. The method utilized ³H-thymidine incorporation, microbead array technology and real time PCR. The six tested cell lines were Namalwa, Jeko-1, Rec-1, Granta-519, DB, and JVM-2.

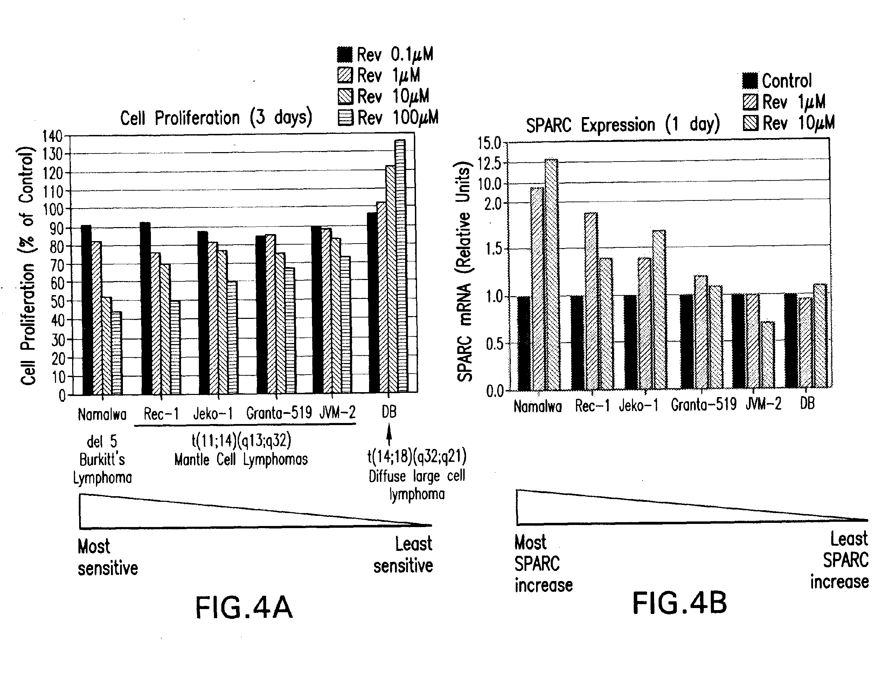

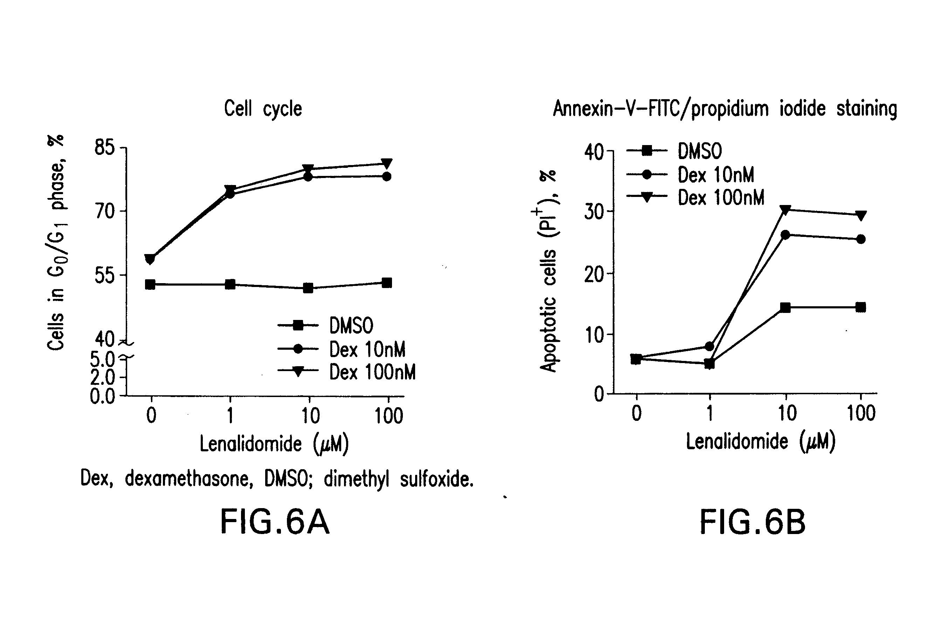

1-oxo-2-(2,6-dioxopiperidin-3-yl)-4-aminoisoindoline demonstrated anti-proliferative activity against several types of NHL cells (FIG. 4A; FIG. 6). The sensitivity of these tested NHL cell lines to 1-oxo-2-(2,6-dioxopiperidin-3-yl)-4-aminoisoindoline is as follows: Namalwa > Rec-1 > Jeko-1 > Granta-519 > JVM-2 > DB cells.

Upon cytogenetic analysis, it was found that Namalwa cells have a 5q deletion. Rec-1, Jeko-1, Granta-519 and JVM-2 cells have a t(11;14)(q13;q32), which is the hallmark for mantle cell lymphoma (MCL) cell lines; and DB cells have the t(14;18)(q32;q21), which is characteristic of follicular lymphoma.

### 6.6 Gene Expression Markers that Predict Sensitivity of Mantle Cell Lymphoma to Treatment with Immunomodulatory Compounds

The finding that expression levels of certain cancer related genes differ between various cancer cell lines led to a more detailed investigation to determine whether certain cancer cell types are more sensitive to specific immunomodulatory compounds than others. Thus, patterns of constitutive (baseline) gene expression that predict sensitivity of NHL tumors to 1-oxo-2-(2,6-dioxopiperidin-3-yl)-4-aminoisoindoline therapy were sought. Baseline expression of specific genes of various NHL cell lines was measured by quantitative real-time polymerase chain reaction (qRT-PCR) using total RNA. The GAPDH gene was used as internal normalizing control (FIG. 3). The results show that cyclin D1 gene is generally over-expressed in MCL cells, as expected based on their characteristic t(11;14) genotype. The level of constitutive cyclin D1 gene expression correlates with the order of sensitivity to 1-oxy-2-(2,6-dioxopiperidin-3-yl)-4-aminoisoindoline: Rec-1 > Jeko-1 > Granta-519 > JVM-2.

### 6.7 Prediction of Treatment Effectiveness in Patients by Measuring Baseline Expression Levels of Cyclin D1 (Reference)

The cell cycle protein cyclin D1, particularly in combination with the cyclin dependent kinase CdK4, stimulates progression through the cell cycle, resulting in an increase in cell proliferation.

The effect of 1-oxo-2-(2,6-dioxopiperidin-3-yl)-4-aminoisoindoline on the expression of cyclin D1 was examined in the six NHL cell types using real-time RT-PCR technology. It was found that a high baseline level of cyclin D1 correlated with sensitivity to 1-oxo-2-(2,6-dioxopiperidin-3-yl)-4-aminoisoindoline (FIG. 3). Thus, cells with high amounts of cyclin D1 expression are more likely to respond to 1-oxo-2-(2,6-dioxopiperidin-3-yl)-4-aminoisoindoline than cells that had low cyclin D1.

Accordingly, the baseline level of cyclin D1 can also be used as markers to predict whether a patient with a given type of NHL will be likely to be effectively treated with an immunomodulatory compound. To predict the likelihood of a successful treatment outcome with a specific immunomodulatory compound, baseline cyclin D1 expression can be monitored by qRT-PCR in lymph node or bone marrow biopsy, or in peripheral blood tumor cells, from patients with NHL and in particular MCL, as a means of predicting which patient will be most likely to benefit from an immunomodulatory compound therapy.

As reference example, a patient with NHL is identified and a lymph node biopsy is taken. Baseline level of cyclin D1 gene expression is measured. The probability of a successful treatment with an immunomodulatory agent is determined by comparing the levels of cyclin D1 with that of cyclin D1 measured in a sample obtained prior to the treatment by an immunomodulatory compound. A patient with a higher baseline cyclin D1 after the treatment is given a high probability of successful treatment with an immunomodulatory compound, and is assigned to a treatment protocol that involves daily oral administration of an immunomodulatory compound.

### 6.8 1-Oxo-2-(2,6-dioxopiperidin-3-yl)-4-aminoisoindoline Administration Increases the Expression of Tumor Suppressor Genes p21cip/kip and SPARC

1-oxo-2-(2,6-dioxopiperidin-3-yl)-4-aminoisoindoline administration increased the expression of tumor suppressor genes, such as *p21*^{*cip*/*kip*} and *SPARC* in several cell lines. The elevation of *SPARC* mRNA correlated with the anti-proliferative effect of 1-oxo-2-(2,6-dioxopiperidin-3-yl)-4-aminoisoindoline and was not seen in the resistant cells, whereas elevated p21 was observed in both sensitive and resistant (DB) cells.

As shown in FIG. 4, 1-oxo-2-(2,6-dioxopiperidin-3-yl)-4-aminoisoindoline increased expression of SPARC mRNA in Non-Hodgkins Lymphoma (NHL) cells lines in a manner that correlates with the antiproliferative activity of 1-oxo-2-(2,6-dioxopiperidin-3-yl)-4-aminoisoindoline against that particular cell line (FIGs. 4A and 4B) The order of sensitivity of the various NHL cell lines to 1-oxo-2-(2,6-dioxopiperidin-3-yl)-4-aminoisoindoline is Namalwa (Burkitt's lymphoma) > Rec-1 > Jeko-1 > Granta-519 > JVM-2 (all Mantle Cell Lymphomas, MCL) > DB (Diffuse Large B Cell Lymphoma, DLBCL. All four of the treated MCL lines contained the characteristic t(11;14) chromosomal translocation that results in overexpression of the cell cycle protein cyclin D1. The DB cell line contained the t(14;18) chromosomal translocation that results in overexpression of the anti-apoptotic protein bc12. This DB cell line actually responded to 1-oxo-2-(2,6-dioxopiperidin-3-yl)-4-aminoisoindoline with an increased rate of cell proliferation. Thus t(14;18) may be a negative prognostic factor in predicting clinical response to 1-oxo-2-(2,6-dioxopiperidin-3-yl)-4-aminoisoindoline. Thus, SPARC gene expression can be used as a biomarker of NHL or MCL tumor response to 1-oxo-2-(2,6-dioxopiperidin-3-yl)-4-aminoisoindoline.

### 6.9 1-Oxo-2-(2,5-dioxopiperidin-3-yl)-4-aminoisoindoline Reduces Production of the Angiogenic Factor VEGF from Sensitive NHL Cells

The effect of 1-oxo-2-(2,6-dioxopiperidin-3-yl)-4-aminoisoindoline on the production of VEGF was measured, as shown in FIG. 7. 1-oxo-2-(2,6-dioxopiperidin-3-yl)-4-aminoisoindoline inhibited the production of the pro-angiogenic growth factor VEGF from anti-proliferation sensitive NHL cells, such as Namalwa, Jeko-1 and Rec-1 cells. This effect on VEGF occurs at concentrations well below levels required for anti-proliferative effects. In contrast, 1-oxo-2-(2,6-dioxopiperidin-3-yl)-4-aminoisoindoline fails to inhibit VEGF from anti-proliferation resistant NHL cells, such as Granta-519 and DB cells. 1-oxo-2-(2,6-dioxopiperidin-3-yl)-4-aminoisoindoline does not inhibit VEGF mRNA expression, implying a post transcriptional inhibitory effect (FIG. 7).

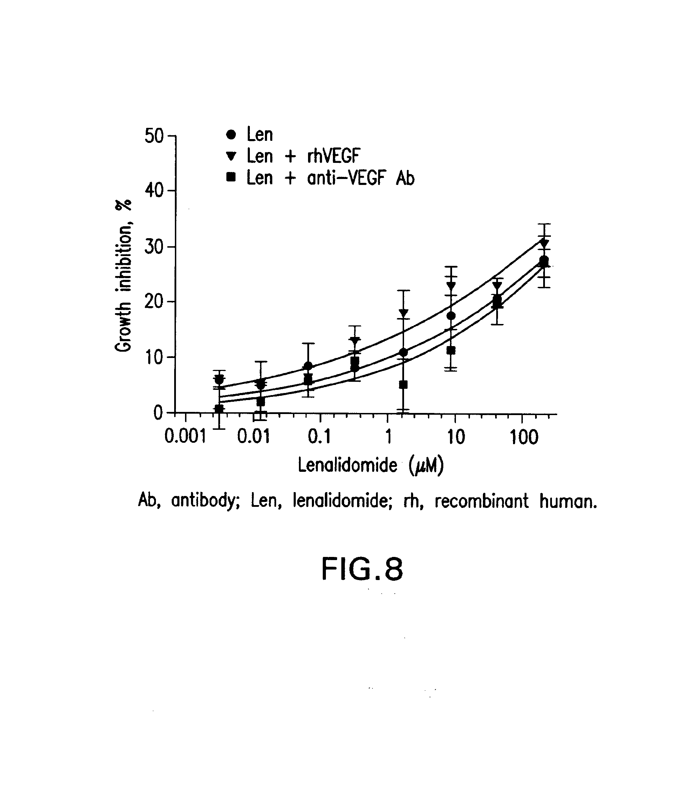

Interestingly, the inhibitory effects of 1-oxo-2-(2,6-dioxopiperidin-3-yl)-4-aminoisoindoline on the proliferation and VEGF production of sensitive NHL cells appear to be independent events, because addition of exogenous recombinant human VEGF (in excess) or neutralizing VEGF antibody does not affect the anti-proliferative activity of 1-oxo-2-(2,6-dioxopiperidin-3-yl)-4-aminoisoindoline (FIG. 8).

### 6.10 RT-PCR Analysis for Gene Expression in NHL Cells Treated with 1-Oxo-2-(2,6-dioxopiperidin-3-yl)-4-aminoisoindoline

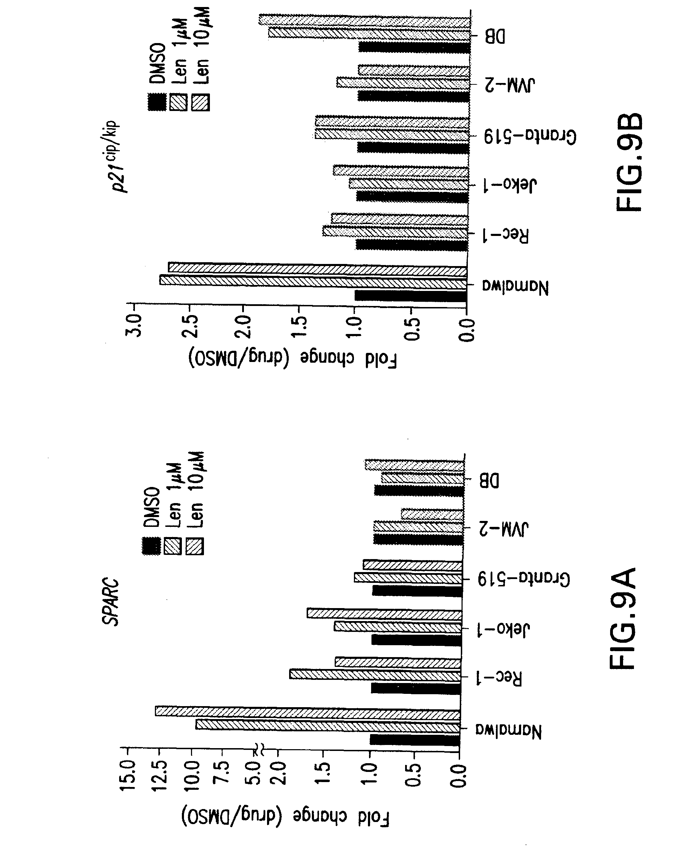

The effect of the administration of 1-oxo-2-(2,6-dioxopiperidin-3-yl)-4-aminoisoindoline in various NHL cells was measured after 24 hours of incubation, using real-time RT-PCR (FIG. 9). The results show that 1-oxo-2-(2,6-dioxopiperidin-3-yl)-4-aminoisoindoline increased the expression of tumor suppressor genes such as p21cip/kipand SPARC in sensitive NHL cells.

### 6.11 Synergy of 1-Oxo-2-(2,6-dioxopiperidin-3-yl)-4-aminoisoindoline and Dexamethasone in Up-regulation of SPARC and p21 Expression in Namalwa Cells

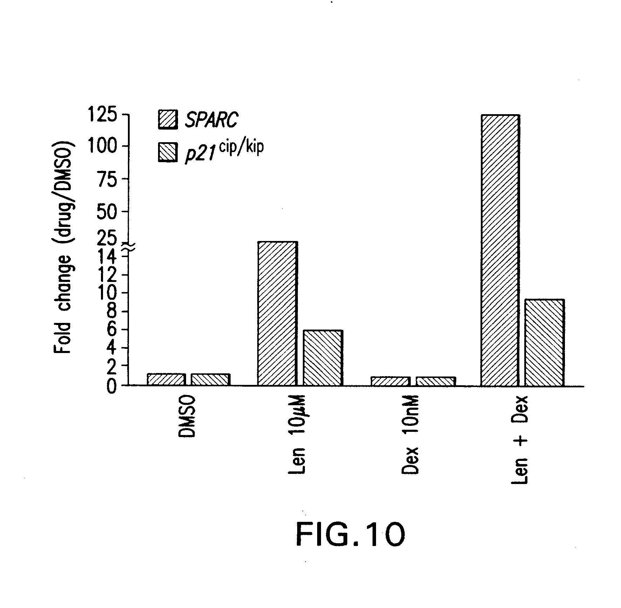

The effect of the administration of 1-oxo-2-(2,6-dioxopiperidin-3-yl)-4-aminoisoindoline in Namalwa cells after 48 hours of drug treatment was measured. The results show that 1-oxo-2-(2,6-dioxopiperidin-3-yl)-4-aminoisoindoline synergized with dexamethasone, upregulating SPARC expression in NHL cells such as Namalwa, Rec-1 and Jeko-1, although dexamethasone administration alone had no effect on SPARC expression. This combination of drugs also significantly up-regulated p21 expression (Figure 10).

### 6.12 Time Kinetic Analysis for Gene Expression in 1-Oxo-2-(2,6-dioxypiperidin-3-yl)-4-aminoisoindoline Treated Namalwa Cells

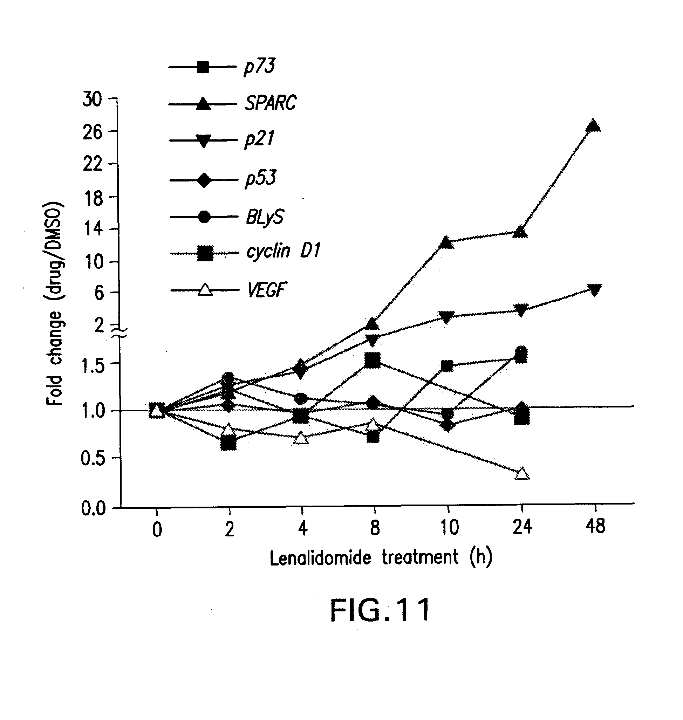

The effect of administration of 1-oxo-2-(2,6-dioxopiperidin-3-yl)-4-aminoisoindoline at various time points from 0 to 48 hours in the fold change in expression of several genes was measured in Namalwa cells (FIG. 11). The 1-oxo-2-(2,6-dioxopiperidin-3-yl)-4-aminoisoindoline treated cells did not exhibit a significant change in expression of several genes involved in angiogenesis, cell proliferation and survival, such as VEGF, p53/p73 (2 members of p53 family transactivating p21 transcritption), BlyS (B lymphocyte stimulator, a member of the TNF ligand family involved in hematopoietic cell survival) and cyclin D1.

### 6.13 SPARC Knockdown Using siRNA Impairs the Antiproliferative Effect of an Immunomodulatory Compound

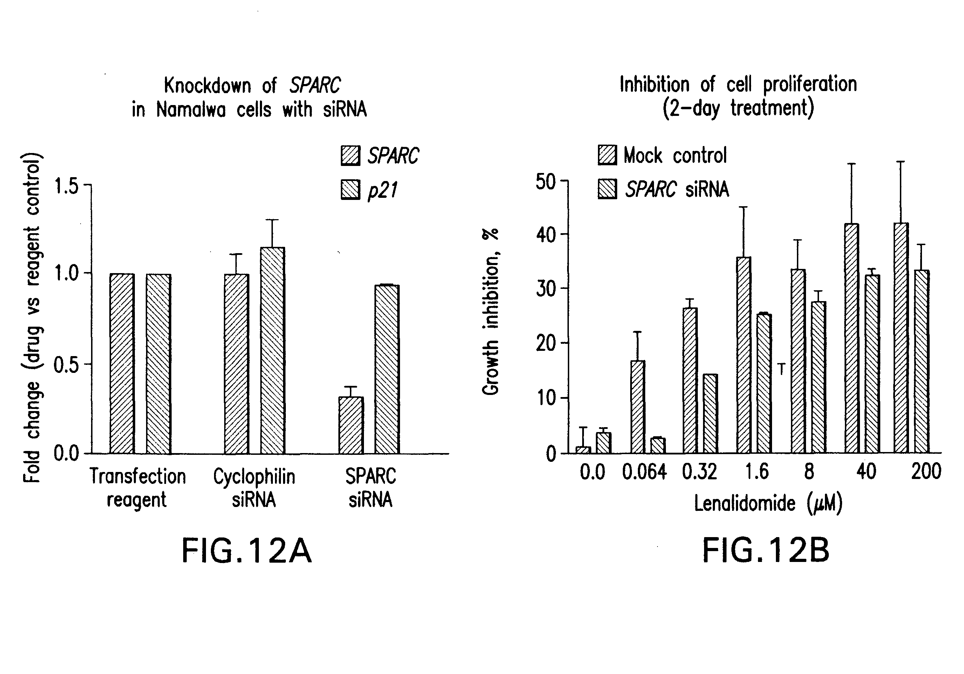

SPARC siRNA was transfected to Namalwa cells to determine the effect of a SPARC knockdown on the antiproliferative effect of 1-oxo-2-(2,6-dioxopiperidin-3-yl)-4-aminoisoindoline in Namalwa cells. The anti-proliferative effect of 1-oxo-2-(2,6-dioxopiperidin-3-yl)-4-aminoisoindoline is weaker after SPARC knockdown compared to mock transfected control cells. Thus, the result shows that the inhibitory effect of 1-oxo-2-(2,6-dioxopiperidin-3-yl)-4-aminoisoindoline on cell proliferation may be impaired, to some extent, by SPARC knockdown (FIG. 12).

## Claims

1. A method of predicting tumor response to treatment in a Non-Hodgkin's Lymphoma (NHL) patient, comprising:
culturing tumor cells obtained from the patient in the presence or absence of an immunomodulatory compound;
measuring SPARC expression in the tumor cells; and
comparing the level of SPARC expression in tumor cells cultured in the presence of an immunomodulatory compound to the level of SPARC expression in tumor cells cultured in the absence of an immunomodulatory compound;
wherein an increased level of SPARC expression in the presence of an immunomodulatory compound indicates the likelihood of an effective patient tumor response to the immunomodulatory compound,
wherein the immunomodulatory compound is 1,3-dioxo-2-(2,6-dioxopiperidine-3-yl)-4-aminoisoindoline.

2. A method of monitoring tumor response to treatment in a Non-Hodgkin's Lymphoma (NHL) patient, comprising:
measuring SPARC expression in a biological sample obtained from the patient;
measuring SPARC expression in a second biological sample obtained from the patient after administration of an immunomodulatory compound to the patient; and
comparing the levels of SPARC expression;
wherein an increased level of SPARC expression after treatment indicates the likelihood of an effective tumor response,
wherein the immunomodulatory compound is 1,3-dioxo-2-(2,6-dioxopiperidine-3-yl)-4-aminoisoindoline.

3. A method for monitoring patient compliance with a drug treatment protocol in the treatment of Non-Hodgkin's lymphoma (NHL), comprising:
measuring the expression level of SPARC in a biological sample obtained from said patient; and
determining if the expression level is increased in the patient sample compared to the expression level in a control untreated sample;
wherein an increased expression indicates patient compliance with said drug treatment protocol,
wherein the drug is 1,3-dioxo-2-(2,6-dioxopiperidine-3-yl)-4-aminoisoindoline.

4. The method of any one of claims 1 to 3, wherein the expression is mRNA expression or protein expression.

5. The method of any one of claims 1 to 4, wherein the expression in the treated sample increases by 1.5x, 2x, 3x, 5x, or more.

6. Use of a kit for predicting the likelihood of an effective treatment of NHL with an immunomodulatory compound, wherein the kit comprises:
a solid support;
a nucleic acid contacting said support, wherein said nucleic acid is complementary to at least 20, 50, 100, 200, 350, or more bases of at least one of p21 mRNA, or SPARC mRNA; and
a means for detecting the expression of said mRNA in a biological sample;
wherein the immunomodulatory compound is 1,3-dioxo-2-(2,6-dioxopiperidine-3-yl)-4-aminoisoindoline.

7. Use of a kit for predicting the likelihood of an effective treatment of NHL with an immunomodulatory compound or for monitoring treatment of NHL with an immunomodulatory compound, wherein the kit comprises:
a solid support; and
a means for detecting the protein expression of at least one of SPARC, and p21 in a biological sample;
wherein the immunomodulatory compound is 1,3-dioxo-2-(2,6-dioxopiperidine-3-yl)-4-aminoisoindoline.

8. The use of claims 6 or 7, wherein the kit employs a dipstick, a membrane, a chip, a disk, a test strip, a filter, a microsphere, a slide, a multiwell plate, or an optical fiber.

9. The use of any one of claims 6 to 8, wherein said solid support comprises a component selected from the group consisting of plastic, silicon, metal, resin, glass, membrane, particle, precipitate, gel, polymer, sheet, sphere, polysaccharide, capillary, film, plate, and slide.

10. The use of any one of claims 6 to 9, wherein said biological sample is selected from the group consisting of a cell lysate, a cell culture, a cell line, a tissue, an organ, an organelle, a biological fluid, a blood sample, a urine sample, and a skin sample.

11. The use of any one of claims 6 to 10, wherein said biological sample is a lymph node biopsy, a bone marrow biopsy, or a sample of peripheral blood tumor cells.

12. The use of claim 10, wherein the tissue is an oral tissue or a gastrointestinal tissue.

## Patentansprüche

1. Verfahren zur Vorhersage des Tumoransprechens auf eine Behandlung eines Non-Hodgkin Lymphom (NLH) Patienten, umfassend:
Kultivieren von vom Patienten in Gegenwart oder Abwesenheit einer immunmodulierenden Verbindung erhaltenen Tumorzellen;
Messen der SPARC-Expression in den Tumorzellen; und
Vergleichen des SPARC-Expressionslevels in Tumorzellen, die in Gegenwart einer immunmodulierenden Verbindung kultiviert sind mit dem SPARC-Expressionslevel in Tumorzellen, die in Abwesenheit einer immunmodulierenden Verbindung kultiviert sind;
wobei ein erhöhter SPARC-Expressionslevel in Gegenwart einer immunmodulierenden Verbindung die Wahrscheinlichkeit eines effektiven Patiententumoransprechens auf die immunmodulierende Verbindung anzeigt;
wobei die immunmodulierende Verbindung 1,3-dioxo-2-(2,6-dioxopiperidin-3-yl)-4-aminoisoindolin ist.

2. Verfahren zum Überwachen des Tumoransprechens auf eine Behandlung eines Non-Hodgkin Lymphom (NLH) Patienten, umfassend:
Messen der SPARC-Expression in einer vom Patienten erhaltenen biologischen Probe;
Messen der SPARC-Expression in einer zweiten, vom Patienten nach der Verabreichung einer immunmodulierenden Verbindung an den Patienten erhaltenen biologischen Probe;
Vergleichen der SPARC-Expressionslevel;
wobei ein nach der Behandlung erhöhter SPARC-Expressionslevel die Wahrscheinlichkeit eines effektiven Tumoransprechens anzeigt;
wobei die immunmodulierende Verbindung 1,3-dioxo-2-(2,6-dioxopiperidin-3-yl)-4-aminoisoindolin ist.

3. Verfahren zum Überwachen der Patientencompliance mit einen Arzneimittelbehandlungsprotokoll bei der Behandlung von Non-Hodgkin Lymphom (NLH), umfassend:
Messen des SPARC-Expressionslevels in einer vom Patienten erhaltenen biologischen Probe; und
Bestimmen, ob der Expressionslevel in der Patientenprobe verglichen zum Expressionslevel in einer unbehandelten Kontrollprobe erhöht ist;
wobei eine erhöhte Expression eine Patientencompliance mit dem Arzneimittelbehandlungsprotokoll anzeigt;
wobei die immunmodulierende Verbindung 1,3-dioxo-2-(2,6-dioxopiperidin-3-yl)-4-aminoisoindolin ist.

4. Verfahren gemäß einem der Ansprüche 1 bis 3, wobei die Expression mRNA-Expression oder Proteinexpression ist.

5. Verfahren gemäß einem der Ansprüche 1 bis 4, wobei die Expression in der behandelten Probe um 1,5x, 2x, 3x, 5x, oder mehr ansteigt.

6. Verwendung eines Kits zur Vorhersage der Wahrscheinlichkeit einer effektiven Behandlung von NHL mit einer immunmdulierenden Verbindung, wobei das Kit aufweist:
Einen festen Träger;
eine Nukleinsäure, die mit dem Träger in Kontakt tritt, wobei die Nukleinsäure komplementär zu mindestens 20, 50, 100, 200, 350, oder mehr Basen von wenigstens einer p21 mRNA oder einer SPARC mRNA ist, und
ein Mittel zum Detektieren der mRNA-Expression in einer biologischen Probe;
wobei die immunmodulierende Verbindung 1,3-dioxo-2-(2,6-dioxopiperidin-3-yl)-4-aminoisoindolin ist.

7. Verwendung eines Kits zur Vorhersage der Wahrscheinlichkeit einer effektiven Behandlung von NHL mit einer immunmodulierender Verbindung oder zum Überwachen der Behandlung von NHL mit einer immunmodulierenden Verbindung, wobei das Kit aufweist:
Einen festen Träger;
ein Mittel zum Detektieren der Proteinexpression von wenigstens einer SPARC oder p21 in einer biologischen Probe;
wobei die immunmodulierende Verbindung 1,3-dioxo-2-(2,6-dioxopiperidin-3-yl)-4-aminoisoindolin ist.

8. Verwendung gemäß Anspruch 6 oder 7, wobei das Kit ein Teststäbchen, eine Membran, einen Chip, eine Disk, einen Teststreifen, einen Filter, eine Mikrokugel, eine Scheibe, eine Multi-Well-Platte, oder eine optische Faser verwendet.

9. Verwendung gemäß einem der Ansprüche 6 bis 8, wobei der feste Träger eine Komponente umfasst, die aus der Gruppe ausgewählt ist, die aus Plastik, Silikon, Metall, Harz, Glas, Membran, Partikel, Präzipitat, Gel, Polymer, Blatt, Kugel, Polysaccharide, Kapillare, Film, Platte, und Scheibe besteht.

10. Verwendung gemäß einem der Ansprüche 6 bis 9, wobei die biologische Probe aus einer Gruppe ausgewählt ist, die aus einem Zelllysat, einer Zellkultur, einer Zelllinie, einem Gewebe, einem Organ, einer Organelle, einem biologischen Fluid, einer Blutprobe, einer Urinprobe, und einer Hautprobe besteht.

11. Verwendung gemäß einem der Ansprüche 6 bis 10 , wobei diese biologische Probe aus einer Lymphknotenbiopsie oder einer Knochenmarkbiopsie stammt, oder eine Probe peripherer Bluttumorzellen ist.

12. Verwendung gemäß Anspruch 10, wobei das Gewebe orales oder gastrointestinales Gewebe ist.

## Revendications

1. Procédé pour prédire la réponse tumorale à un traitement chez un patient souffrant d'un lymphome non-hodgkinien (LNH), comprenant :
- la culture de cellules tumorales obtenues à partir du patient en présence ou en l'absence d'un composé d'immunomodulation ;
- la mesure de l'expression de SPARC dans les cellules tumorales ; et
- la comparaison du niveau de l'expression de SPARC dans des cellules tumorales cultivées en présence d'un composé d'immunomodulation au niveau de l'expression de SPARC dans des cellules tumorales cultivées en l'absence d'un composé d'immunomodulation ;
un niveau augmenté de l'expression de SPARC en présence d'un composé d'immunomodulation indiquant la probabilité d'une réponse tumorale efficace du patient au composé d'immunomodulation,
le composé d'immunomodulation étant la 1,3-dioxo-2-(2,6-dioxopipéridin-3-yl)-4-amino-iso-indoline.

2. Procédé pour surveiller la réponse tumorale à un traitement chez un patient souffrant d'un lymphome non-hodgkinien (LNH), comprenant :
- la mesure de l'expression de SPARC dans un échantillon biologique obtenu à partir du patient ;
- la mesure de l'expression de SPARC dans un deuxième échantillon biologique obtenu à partir du patient après l'administration d'un composé d'immunomodulation au patient ; et
- la comparaison des niveaux de l'expression de SPARC ;
un niveau augmenté de l'expression de SPARC après le traitement indiquant la probabilité d'une réponse tumorale efficace,
le composé d'immunomodulation étant la 1,3-dioxo-2-(2,6-dioxopipéridin-3-yl)-4-amino-iso-indoline.

3. Procédé pour surveiller l'observance d'un patient à un protocole de traitement médicamenteux dans le traitement d'un lymphome non-hodgkinien (LNH), comprenant :
- la mesure du niveau de l'expression de SPARC dans un échantillon biologique obtenu à partir dudit patient ; et
- la détermination de l'augmentation ou non du niveau d'expression dans l'échantillon du patient comparativement au niveau d'expression dans un échantillon témoin non traité ;
une expression augmentée indiquant l'observance du patient audit protocole de traitement médicamenteux,
le médicament étant la 1,3-dioxo-2-(2,6-dioxopipéridin-3-yl)-4-amino-iso-indoline.

4. Procédé selon l'une quelconque des revendications 1 à 3, l'expression étant l'expression d'ARNm ou l'expression protéinique.

5. Procédé selon l'une quelconque des revendications 1 à 4, l'expression dans l'échantillon traité augmentant d'un facteur 1.5, 2, 3, 5 ou plus.

6. Utilisation d'un kit pour prédire la probabilité d'un traitement efficace du LNH par un composé d'immunomodulation, le kit comprenant
- un support solide ;
- un acide nucléique en contact avec ledit support, ledit acide nucléique étant complémentaire à au moins 20, 50, 100, 200, 350 bases ou plus d'au moins un ARNm de p21 ou ARNm de SPARC ; et
- un moyen pour détecter l'expression dudit ARNm dans un échantillon biologique ;
le composé d'immunomodulation étant la 1,3-dioxo-2-(2,6-dioxopipéridin-3-yl)-4-amino-iso-indoline.

7. Utilisation d'un kit pour prédire la probabilité d'un traitement efficace du LNH par un composé d'immunomodulation, ou pour surveiller le traitement du LNH par un composé d'immunomodulation, le kit comprenant
- un support solide ; et
- un moyen pour détecter l'expression protéinique d'au moins un parmi SPARC et p21 dans un échantillon biologique ;
le composé d'immunomodulation étant la 1,3-dioxo-2-(2,6-dioxopipéridin-3-yl)-4-amino-iso-indoline.

8. Utilisation selon les revendications 6 ou 7, le kit utilisant une jauge graduée, une membrane, une puce, un disque, une bandelette réactive, un filtre, une microsphère, une lame, une plaque à cuvettes multiples ou une fibre optique.

9. Utilisation selon l'une quelconque des revendications 6 à 8, ledit support solide comprenant un constituant choisi dans le groupe constitué par le plastique, la silicone, le métal, la résine, le verre, une membrane, une particule, un précipité, un gel, un polymère, une feuille, une sphère, un polysaccharide, un capillaire, un film, une plaque et une lame.

10. Utilisation selon l'une quelconque des revendications 6 à 9, ledit échantillon biologique étant choisi dans le groupe constitué par un lysat cellulaire, une culture cellulaire, une lignée cellulaire, un tissu, un organe, une organelle, un fluide biologique, un échantillon de sang, un échantillon d'urine et un échantillon de la peau.

11. Utilisation selon l'une quelconque des revendications 6 à 10, ledit échantillon biologique étant une biopsie de ganglion lymphatique, une biopsie de moelle osseuse ou un échantillon de cellules tumorales du sang périphérique.

12. Utilisation selon la revendication 10, le tissu étant un tissu oral ou un tissu gastro-intestinal.
